# EUROPEAN PATENT APPLICATION

(11) **EP 3 901 152 A1**
(43) Date of publication of application: **27.10.2021**
(21) Application number: 20170961.5
(22) Date of filing: 23.04.2020
(51) Int. Cl.: C07D 471/04, C07D 401/04, C07D 405/12, C07D 405/14, C07D 487/04, A61K 31/437, C07D 473/00, A61P 25/00

(54) **KV3 ENHANCERS FOR THE TREATMENT OF COGNITIVE DISORDERS**

(71) Applicant: F. Hoffmann-La Roche AG, 4070 Basel (CH)
(72) Inventor: The designation of the inventor has not yet been filed
(74) Representative: Neuhaus, Christian Michel

(57) **Abstract**

The invention provides new heterocyclic compounds having the general formula (I) 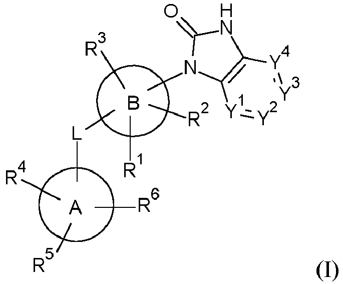 wherein A, B, L, Y¹ to Y⁴ and R¹ to R⁶ are as described herein.

## Description

### Field of the Invention

The present invention relates to new organic compounds useful as Kv3 enhancers (or positive modulators), their manufacture, pharmaceutical compositions comprising said compounds and their use as medicaments for the therapeutic and/or prophylactic treatment of diseases associated with Kv3, such as neurodevelopmental disorders like autism and fragile X syndrome, epilepsy, intellectual disability and cognitive impairment, ataxia, depression, schizophrenia, attention deficit hyperactivity disorder and sensory processing disorders (auditory sensory processing disorders).

### Background of the Invention

The potassium channel Kv3 is a protein with four subtypes that in humans is encoded by the gene KCNC1, KCNC2, KCNC3 and KCNC4.

The KCNC channel family responds to changes in membrane voltage and opens to let potassium ions flow out of cell membranes. KCNC1 and KCNC2 are expressed primarily on GABAergic neurons in the brain, and their activation under natural conditions leads to the repolarization of action potentials. Kv3 channels are currently being investigated because of their unique distribution and their potential role as primary regulators of inhibitory neurons in many CNS and PNS pathways (Kaczmarek, L. K. & Zhang, Y. Kv3 Channels: Enablers of Rapid Firing, Neurotransmitter Release, and Neuronal Endurance. Physiol. Rev. 97, 1431-1468 (2017*)).*

Kv3 enhancers are emerging as promising therapeutic agents for the treatment of sensory processing deficits linked to cognition symptoms as well as disease-modifying agents in Schizophrenia through a non-dopaminergic approach.

The normalization of sensory processing by Kv3.1/3.2 enhancers was also described in fragile X syndrome models (Ethridge, L. E. et al. Neural synchronization deficits linked to cortical hyper-excitability and auditory hypersensitivity in fragile X syndrome syndrome. Mol. Autism 8, 22 (2017)) as well as in auditory sensory processing (Chambers, A. R. et al. Pharmacological modulation of Kv3.1 mitigates auditory midbrain temporal processing deficits following auditory nerve damage. Sci. Rep. 7, 17496 (2017); Hutchison, J. et al. Early Clinical Evaluation of AUT00206, a Novel Selective Kv3 Channel Modulator for the Treatment of Schizophrenia. in NEUROPSYCHOPHARMACOLOGY vol. 41 S386-S386 (NATURE PUBLISHING GROUP MACMILLAN BUILDING, 4 CRINAN ST, LONDON N1 9XW, ENGLAND, 2016); Anderson, L. A. et al. Increased spontaneous firing rates in auditory midbrain following noise exposure are specifically abolished by a Kv3 channel modulator. Hear. Res. 365, 77-89 (2018).

Moreover, KCNC1 has been genetically linked to epilepsy (Muona, M. et al. A recurrent de novo mutation in KCNC1 causes progressive myoclonus epilepsy. Nat. Genet. 47, 39-46 (2015)), ataxia (Figueroa, K. P. et al. KCNC3: phenotype, mutations, channel biophysics-a study of 260 familial ataxia patients. Hum. Mutat. 31, 191-196 (2010); Nascimento, F. A. & Andrade, D. M. Myoclonus epilepsy and ataxia due to potassium channel mutation (MEAK) is caused by heterozygous KCNC1 mutations. Epileptic Disord. 18, 135-138 (2016) and ADHD (Lesch, K.-P. et al. Molecular genetics of adult ADHD: converging evidence from genome-wide association and extended pedigree linkage studies. J. Neural Transm. 115, 1573-1585 (2008)).

Therefore, modulating the Kv3 activity is a promising strategy for the treatment or prevention of diseases associated with Kv3, such as neurodevelopmental disorders like autism and fragile X syndrome, epilepsy, intellectual disability and cognitive impairment, ataxia, depression, schizophrenia, attention deficit hyperactivity disorder and sensory processing disorders (e.g., auditory sensory processing disorders).

It is, therefore, an object of this invention to provide Kv3.1/Kv3.2 enhancers useful for the treatment or prevention or amelioration of Kv3 mediated diseases and disorders, such as neurodevelopmental disorders like autism and fragile X syndrome, epilepsy, intellectual disability and cognitive impairment, ataxia, depression, schizophrenia, attention deficit hyperactivity disorder and sensory processing disorders (e.g., auditory sensory processing disorders).

### Summary of the Invention

In a first aspect, the present invention provides a compound of formula (I), or a pharmaceutically acceptable salt thereof, 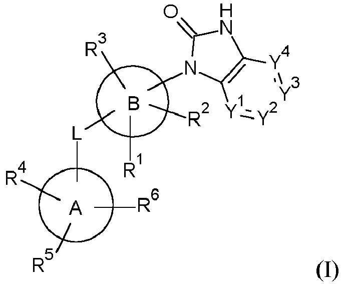 wherein A, B, L, Y¹ to Y⁴ and R¹ to R⁶ are as described herein.

In one aspect, the present invention provides a process of manufacturing the compounds of formula (I) described herein, comprising reacting a diamine of formula 1 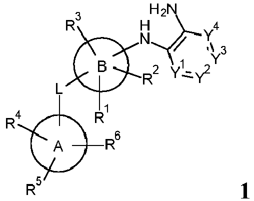 with a coupling agent, such as CDI or triphosgene, to form said compound of formula (I).

In a further aspect, the present invention provides a compound of formula (I) as described herein, when manufactured according to the processes described herein.

In a further aspect, the present invention provides a compound of formula (I) as described herein, for use as therapeutically active substance.

In a further aspect, the present invention provides a pharmaceutical composition comprising a compound of formula (I) as described herein and a therapeutically inert carrier.

In a further aspect, the present invention provides a compound of formula (I) as described herein, or a pharmaceutically acceptable salt thereof, or a pharmaceutical composition comprising said compound of formula (I) or a pharmaceutically acceptable salt thereof, for use in the treatment or prophylaxis of Kv3 mediated diseases and disorders.

### Detailed Description of the Invention

### Definitions

Features, integers, characteristics, compounds, chemical moieties or groups described in conjunction with a particular aspect, embodiment or example of the invention are to be understood to be applicable to any other aspect, embodiment or example described herein, unless incompatible therewith. All of the features disclosed in this specification (including any accompanying claims, abstract and drawings), and/or all of the steps of any method or process so disclosed, may be combined in any combination, except combinations where at least some of such features and/or steps are mutually exclusive. The invention is not restricted to the details of any foregoing embodiments. The invention extends to any novel one, or any novel combination, of the features disclosed in this specification (including any accompanying claims, abstract and drawings), or to any novel one, or any novel combination, of the steps of any method or process so disclosed.

The term "alkyl" refers to a mono- or multivalent, e.g., a mono- or bivalent, linear or branched saturated hydrocarbon group of 1 to 12 carbon atoms. In some preferred embodiments, the alkyl group contains 1 to 6 carbon atoms ("C₁₋₆-alkyl"), e.g., 1, 2, 3, 4, 5, or 6 carbon atoms. In other embodiments, the alkyl group contains 1 to 3 carbon atoms, e.g., 1, 2 or 3 carbon atoms. Some non-limiting examples of alkyl include methyl, ethyl, propyl, 2-propyl (isopropyl), n-butyl, isobutyl, sec-butyl, tert-butyl, and 2,2-dimethylpropyl. A particularly preferred, yet non-limiting example of alkyl is methyl.

The term "alkoxy" refers to an alkyl group, as previously defined, attached to the parent molecular moiety via an oxygen atom. Unless otherwise specified, the alkoxy group contains 1 to 12 carbon atoms. In some preferred embodiments, the alkoxy group contains 1 to 6 carbon atoms ("C₁₋₆-alkoxy"). In other embodiments, the alkoxy group contains 1 to 4 carbon atoms. In still other embodiments, the alkoxy group contains 1 to 3 carbon atoms. Some non-limiting examples of alkoxy groups include methoxy, ethoxy, n-propoxy, isopropoxy, n-butoxy, isobutoxy and tert-butoxy. A particularly preferred, yet non-limiting example of alkoxy is methoxy.

The term "halogen" or "halo" refers to fluoro (F), chloro (Cl), bromo (Br), or iodo (I). Preferably, the term "halogen" or "halo" refers to fluoro (F), chloro (Cl) or bromo (Br). Particularly preferred, yet non-limiting examples of "halogen" or "halo" are fluoro (F) and chloro (Cl).

The term "cycloalkyl" as used herein refers to a saturated or partly unsaturated monocyclic or polycyclic hydrocarbon group of 3 to 10 ring carbon atoms ("C₃-C₁₀-cycloalkyl"). In some preferred embodiments, the cycloalkyl group is a saturated or partly unsaturated monocyclic hydrocarbon group of 3 to 8 ring carbon atoms. In some preferred embodiments, the cycloalkyl group is a saturated or partly unsaturated bicyclic hydrocarbon group of 3 to 8 ring carbon atoms. "Bicyclic hydrocarbon group" refers to cycloalkyl moieties consisting of two saturated or partly unsaturated carbocycles having two carbon atoms in common, i.e., the bridge separating the two rings is either a single bond or a chain of one or two ring atoms, and to spirocyclic moieties, i.e., the two rings are connected via one common ring atom. In some embodiments, "polycyclic hydrocarbon" refers to C₃-C₁₀-polycyclic bridged units such as cubane or bicyclo[1.1.1]pentane. In some embodiments, the cycloalkyl group is a saturated monocyclic hydrocarbon group of 3 to 6 ring carbon atoms, e.g., of 3, 4, 5 or 6 carbon atoms. Some non-limiting examples of cycloalkyl include cyclopropyl, cyclobutyl, cyclopentyl, cyclohexyl, cycloheptyl and bicyclo[1.1.1]pentane. A particularly preferred example of cycloalkyl is cyclopropyl. A further particularly preferred example of cycloalkyl is bicyclo[1.1.1]pentane.

The terms "heterocyclyl" and "heterocycloalkyl" are used herein interchangeably and refer to a saturated or partly unsaturated mono- or bicyclic, preferably monocyclic ring system of 3 to 10 ring atoms, preferably 3 to 8 ring atoms, wherein 1, 2, or 3 of said ring atoms are heteroatoms selected from N, O and S, the remaining ring atoms being carbon. Preferably, 1 to 2 of said ring atoms are selected from N and O, the remaining ring atoms being carbon. "Bicyclic heterocyclyl" refers to heterocyclic moieties consisting of two cycles having two ring atoms in common, i.e., the bridge separating the two rings is either a single bond or a chain of one or two ring atoms, and to spirocyclic moieties, i.e., the two rings are connected via one common ring atom. Some non-limiting examples of monocyclic heterocyclyl groups include azetidin-3-yl, azetidin-2-yl, oxetan-3-yl, oxetan-2-yl, 1-piperidyl, 2-piperidyl, 3-piperidyl, 4-piperidyl, 2-oxopyrrolidin-1-yl, 2-oxopyrrolidin-3-yl, 5-oxopyrrolidin-2-yl, 5-oxopyrrolidin-3-yl, 2-oxo-1-piperidyl, 2-oxo-3-piperidyl, 2-oxo-4-piperidyl, 6-oxo-2-piperidyl, 6-oxo-3-piperidyl, morpholino, morpholin-2-yl and morpholin-3-yl. Some non-limiting examples of bicyclic heterocyclyl groups include 5-oxaspiro[2.4]hept-6-ene and 5-thiaspiro[2.4]hept-6-ene.

The term "aryl" refers to a monocyclic, bicyclic, or tricyclic carbocyclic ring system having a total of 6 to 14 ring members ("C₆-C₁₄-aryl"), preferably, 6 to 12 ring members, and more preferably 6 to 10 ring members, and wherein at least one ring in the system is aromatic. Some non-limiting examples of aryl include phenyl and 9H-fluorenyl (e.g. 9H-fluoren-9-yl). A particularly preferred, yet non-limiting example of aryl is phenyl.

The term "heteroaryl" refers to a mono- or multivalent, monocyclic or bicyclic ring system having a total of 5 to 14 ring members, preferably, 5 to 12 ring members, and more preferably 5 to 10 ring members, wherein at least one ring in the system is aromatic, and at least one ring in the system contains one or more heteroatoms. Preferably, "heteroaryl" refers to a 5-10 membered heteroaryl comprising 1, 2, 3 or 4 heteroatoms independently selected from O, S and N. Most preferably, "heteroaryl" refers to a 5-10 membered heteroaryl comprising 1 to 2 heteroatoms independently selected from O, S and N. A preferred, yet non-limiting example of heteroaryl includes pyridyl.

The term "hydroxy" refers to an -OH group.

The term "cyano" refers to a -CN (nitrile) group.

The term "haloalkyl" refers to an alkyl group, wherein at least one of the hydrogen atoms of the alkyl group has been replaced by a halogen atom, preferably fluoro. Preferably, "haloalkyl" refers to an alkyl group wherein 1, 2 or 3 hydrogen atoms of the alkyl group have been replaced by a halogen atom, most preferably fluoro. Particularly preferred, yet non-limiting examples of haloalkyl are trifluoromethyl (CF₃) and trifluoroethyl (e.g. 2,2,2-trifluoroethyl).

The term "hydroxyalkyl" refers to an alkyl group, wherein at least one of the hydrogen atoms of the alkyl group has been replaced by a hydroxy group. Preferably, "hydroxyalkyl" refers to an alkyl group wherein 1, 2 or 3 hydrogen atoms of the alkyl group have been replaced by a hydroxy group. Particularly preferred, yet non-limiting examples of hydroxyalkyl are hydroxymethyl and hydroxyethyl.

The term "alkoxyalkyl" refers to an alkyl group, wherein at least one of the hydrogen atoms of the alkyl group has been replaced by an alkoxy group. Preferably, "alkoxyalkyl" refers to an alkyl group wherein 1, 2 or 3 hydrogen atoms of the alkyl group have been replaced by an alkoxy group. Particularly preferred, yet non-limiting examples of alkoxyalkyl are methoxymethyl, ethoxymethyl, methoxyethyl and ethoxyethyl.

The term "haloalkoxy" refers to an alkoxy group, wherein at least one of the hydrogen atoms of the alkoxy group has been replaced by a halogen atom, preferably fluoro. Preferably, "haloalkoxy" refers to an alkoxy group wherein 1, 2 or 3 hydrogen atoms of the alkoxy group have been replaced by a halogen atom, most preferably fluoro. A particularly preferred, yet non-limiting example of haloalkoxy is trifluoromethoxy (-OCF₃).

The term "pharmaceutically acceptable salt" refers to those salts which retain the biological effectiveness and properties of the free bases or free acids, which are not biologically or otherwise undesirable. The salts are formed with inorganic acids such as hydrochloric acid, hydrobromic acid, sulfuric acid, nitric acid, phosphoric acid and the like, in particular hydrochloric acid, and organic acids such as acetic acid, propionic acid, glycolic acid, pyruvic acid, oxalic acid, maleic acid, malonic acid, succinic acid, fumaric acid, tartaric acid, citric acid, benzoic acid, cinnamic acid, mandelic acid, methanesulfonic acid, ethanesulfonic acid, p-toluenesulfonic acid, salicylic acid, N-acetylcystein and the like. In addition these salts may be prepared by addition of an inorganic base or an organic base to the free acid. Salts derived from an inorganic base include, but are not limited to, the sodium, potassium, lithium, ammonium, calcium, magnesium salts and the like. Salts derived from organic bases include, but are not limited to salts of primary, secondary, and tertiary amines, substituted amines including naturally occurring substituted amines, cyclic amines and basic ion exchange resins, such as isopropylamine, trimethylamine, diethylamine, triethylamine, tripropylamine, ethanolamine, lysine, arginine, N-ethylpiperidine, piperidine, polyimine resins and the like. Particular pharmaceutically acceptable salts of compounds of formula (I) are hydrochloride salts.

The compounds of formula (I) can contain several asymmetric centers and can be present in the form of optically pure enantiomers, mixtures of enantiomers such as, for example, racemates, optically pure diastereioisomers, mixtures of diastereoisomers, diastereoisomeric racemates or mixtures of diastereoisomeric racemates. In a preferred embodiment, the compound of formula (I) according to the invention is a *cis*-enantiomer of formula (Ia) or (Ib), respectively, as described herein.

According to the Cahn-Ingold-Prelog Convention, the asymmetric carbon atom can be of the "R" or "S" configuration.

The term "treatment" as used herein includes: (1) inhibiting the state, disorder or condition (e.g. arresting, reducing or delaying the development of the disease, or a relapse thereof in case of maintenance treatment, of at least one clinical or subclinical symptom thereof); and/or (2) relieving the condition (i.e., causing regression of the state, disorder or condition or at least one of its clinical or subclinical symptoms). The benefit to a patient to be treated is either statistically significant or at least perceptible to the patient or to the physician. However, it will be appreciated that when a medicament is administered to a patient to treat a disease, the outcome may not always be effective treatment.

The term "prophylaxis" as used herein includes: preventing or delaying the appearance of clinical symptoms of the state, disorder or condition developing in a mammal and especially a human that may be afflicted with or predisposed to the state, disorder or condition but does not yet experience or display clinical or subclinical symptoms of the state, disorder or condition.

The term "mammal" as used herein includes both humans and non-humans and includes but is not limited to humans, non-human primates, canines, felines, murines, bovines, equines, and porcines. In a particularly preferred embodiment, the term "mammal" refers to humans.

The term "neurodevelopmental disorder" refers to a group of disorders that affect the development of the nervous system, leading to abnormal brain function, which may affect emotion, learning ability, self-control, and memory. Some non-limiting examples of neurodevelopmental disorder include, but are not limited to, autism, fragile X syndrome, epilepsy, intellectual disability, cognitive impairment, ataxia, depression, schizophrenia, attention deficit hyperactivity disorder and sensory processing disorders (e.g., auditory sensory processing disorders).

### Compounds of the Invention

In a first aspect (A1), the present invention provides a compound of formula (I) 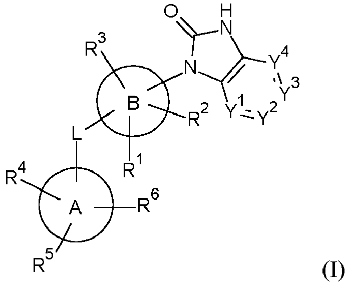 or a pharmaceutically acceptable salt thereof, wherein:
A and B are each independently selected from C₆-C₁₄-aryl, 5- to 14-membered heteroaryl, C₃-C₁₀-cycloalkyl and 3- to 14-membered heterocyclyl;
L is selected from -CH₂-, -O-, -NH-, -S-, -SO- and -SO₂-;
Y¹, Y², Y³ and Y⁴ are each independently selected from CH, N, C-halogen, C-cyano, C-OH, C-C₁-C₆-alkyl, C-halo-C₁-C₆-alkyl, C-hydroxy-C₁-C₆-alkyl, C-C₁-C₆-alkoxy-C₁-C₆-alkyl, C-C₁-C₆-alkoxy, C-halo-C₁-C₆-alkoxy, C-S-C₁-C₆-alkyl, C-SO-C₁-C₆-alkyl, C-SO₂-C₁-C₆-alkyl, C-C₃-C₁₀-cycloalkyl and C-(3- to 14-membered heterocyclyl);
   (i) R¹, R², R³, R⁴, R⁵ and R⁶ are each independently selected from hydrogen, halogen, cyano, hydroxy, C₁-C₆-alkyl, halo-C₁-C₆-alkyl, hydroxy-C₁-C₆-alkyl, C₁-C₆-alkoxy-C₁-C₆-alkyl, C₁-C₆-alkoxy, halo-C₁-C₆-alkoxy, -S-C₁-C₆-alkyl, -SO-C₁-C₆-alkyl, - SO₂-C₁-C₆-alkyl, C₃-C₁₀-cycloalkyl and 3- to 14-membered heterocyclyl; or
   (ii) R¹, R², R³, and R⁶ are each independently selected from hydrogen, halogen, cyano, hydroxy, C₁-C₆-alkyl, halo-C₁-C₆-alkyl, hydroxy-C₁-C₆-alkyl, C₁-C₆-alkoxy-C₁-C₆-alkyl, C₁-C₆-alkoxy, halo-C₁-C₆-alkoxy, -S-C₁-C₆-alkyl, -SO-C₁-C₆-alkyl, -SO₂-C₁-C₆-alkyl, C₃-C₁₀-cycloalkyl and 3- to 14-membered heterocyclyl; and R⁴ and R⁵, taken together with the atoms to which they are attached, form a 3- to 14-membered heterocycle or a C₃-C₁₀-cycloalkyl.

In one embodiment, the present invention provides a compound of formula (I), or a pharmaceutically acceptable salt thereof, wherein A is selected from C₆-C₁₄-aryl and 5- to 14-membered heteroaryl.

In a preferred embodiment, the present invention provides a compound of formula (I), or a pharmaceutically acceptable salt thereof, wherein A is C₆-C₁₄-aryl.

In a particularly preferred embodiment, the present invention provides a compound of formula (I), or a pharmaceutically acceptable salt thereof, wherein A is phenyl.

In one embodiment, the present invention provides a compound of formula (I), or a pharmaceutically acceptable salt thereof, wherein B is selected from C₆-C₁₄-aryl, 5- to 14-membered heteroaryl and C₃-C₁₀-cycloalkyl.

In a preferred embodiment, the present invention provides a compound of formula (I), or a pharmaceutically acceptable salt thereof, wherein B is 5- to 14-membered heteroaryl.

In a particularly preferred embodiment, the present invention provides a compound of formula (I), or a pharmaceutically acceptable salt thereof, wherein B is pyridyl.

In one embodiment, the present invention provides a compound of formula (I), or a pharmaceutically acceptable salt thereof, wherein B is C₃-C₁₀-cycloalkyl.

In a preferred embodiment, the present invention provides a compound of formula (I), or a pharmaceutically acceptable salt thereof, wherein B is C₃-C₁₀-cycloalkyl, wherein said C₃-C₁₀-cycloalkyl is a polycyclic bridged C₃-C₁₀-cycloalkyl.

In a particularly preferred embodiment, the present invention provides a compound of formula (I), or a pharmaceutically acceptable salt thereof, wherein B is C₃-C₁₀-cycloalkyl, wherein said cycloalkyl is a polycyclic bridged C₃-C₁₀-cycloalkyl, wherein said polycyclic bridged C₃-C₁₀-cycloalkyl is cubane or bicyclo[1.1.1]pentane, in particular wherein said polycyclic bridged C₃-C₁₀-cycloalkyl is bicyclo[1.1.1]pentane.

In a particularly preferred embodiment, the present invention provides a compound of formula (I), or a pharmaceutically acceptable salt thereof, wherein L is -O-.

In a particularly preferred embodiment, the present invention provides a compound of formula (I), or a pharmaceutically acceptable salt thereof, wherein Y¹ is CH or N.

In one embodiment, the present invention provides a compound of formula (I), or a pharmaceutically acceptable salt thereof, wherein Y² is CH or N.

In a particularly preferred embodiment, the present invention provides a compound of formula (I), or a pharmaceutically acceptable salt thereof, wherein Y² is CH.

In one embodiment, the present invention provides a compound of formula (I), or a pharmaceutically acceptable salt thereof, wherein Y³ is selected from CH, N, C-halogen, C-C₁-C₆-alkyl and C-halo-C₁-C₆-alkyl.

In a preferred embodiment, the present invention provides a compound of formula (I), or a pharmaceutically acceptable salt thereof, wherein Y³ is selected from CH, N, C-halogen and C-C₁-C₆-alkyl.

In a particularly preferred embodiment, the present invention provides a compound of formula (I), or a pharmaceutically acceptable salt thereof, wherein Y³ is selected from CH, N, C-F and C-methyl.

In one embodiment, the present invention provides a compound of formula (I), or a pharmaceutically acceptable salt thereof, wherein Y⁴ is CH or N.

In a particularly preferred embodiment, the present invention provides a compound of formula (I), or a pharmaceutically acceptable salt thereof, wherein Y⁴ is CH.

In one embodiment, the present invention provides a compound of formula (I), or a pharmaceutically acceptable salt thereof, wherein R¹ is selected from hydrogen, halogen and C₁-C₆-alkyl.

In a particularly preferred embodiment, the present invention provides a compound of formula (I), or a pharmaceutically acceptable salt thereof, wherein R¹ is hydrogen.

In a particularly preferred embodiment, the present invention provides a compound of formula (I), or a pharmaceutically acceptable salt thereof, wherein R² is hydrogen.

In a particularly preferred embodiment, the present invention provides a compound of formula (I), or a pharmaceutically acceptable salt thereof, wherein R³ is hydrogen.

In one embodiment, the present invention provides a compound of formula (I), or a pharmaceutically acceptable salt thereof, wherein R⁴ is selected from C₁-C₆-alkyl, halo-C₁-C₆-alkyl and C₁-C₆-alkoxy.

In a preferred embodiment, the present invention provides a compound of formula (I), or a pharmaceutically acceptable salt thereof, wherein R⁴ is C₁-C₆-alkyl.

In a particularly preferred embodiment, the present invention provides a compound of formula (I), or a pharmaceutically acceptable salt thereof, wherein R⁴ is methyl.

In one embodiment, the present invention provides a compound of formula (I), or a pharmaceutically acceptable salt thereof, wherein R⁵ is selected from halogen, cyano, C₁-C₆-alkoxy and halo-C₁-C₆-alkoxy.

In a preferred embodiment, the present invention provides a compound of formula (I), or a pharmaceutically acceptable salt thereof, wherein R⁵ is C₁-C₆-alkoxy.

In a particularly preferred embodiment, the present invention provides a compound of formula (I), or a pharmaceutically acceptable salt thereof, wherein R⁵ is methoxy.

In one embodiment, the present invention provides a compound of formula (I), or a pharmaceutically acceptable salt thereof, wherein R⁴ and R⁵, taken together with the atoms to which they are attached, form a 3- to 14-membered heterocycle.

In a particularly preferred embodiment, the present invention provides a compound of formula (I), or a pharmaceutically acceptable salt thereof, wherein R⁴ and R⁵, taken together with the atoms to which they are attached, form a 5-oxaspiro[2.4]hept-6-ene or a 5-thiaspiro[2.4]hept-6-ene ring.

In one embodiment, the present invention provides a compound of formula (I), or a pharmaceutically acceptable salt thereof, wherein R⁶ is hydrogen or C₁-C₆-alkyl.

In a particularly preferred embodiment, the present invention provides a compound of formula (I), or a pharmaceutically acceptable salt thereof, wherein R⁶ is hydrogen.

In one embodiment, the present invention provides a compound of formula (I), or a pharmaceutically acceptable salt thereof, wherein:
A is selected from C₆-C₁₄-aryl and 5- to 14-membered heteroaryl;
B is selected from C₆-C₁₄-aryl, 5- to 14-membered heteroaryl and C₃-C₁₀-cycloalkyl;
L is -O-;
Y¹, Y² and Y⁴ are each independently selected from CH and N;
Y³ is selected from CH, N, C-halogen, C-C₁-C₆-alkyl and C-halo-C₁-C₆-alkyl;
R¹ is selected from hydrogen, halogen and C₁-C₆-alkyl;
R² and R³ are each hydrogen;
R⁴ is selected from C₁-C₆-alkyl, halo-C₁-C₆-alkyl and C₁-C₆-alkoxy; and
R⁵ is selected from halogen, cyano, C₁-C₆-alkoxy and halo-C₁-C₆-alkoxy; or
R⁴ and R⁵, taken together with the atoms to which they are attached, form a 3- to 14-membered heterocycle; and
R⁶ is hydrogen or C₁-C₆-alkyl.

In a preferred embodiment, the present invention provides a compound of formula (I), or a pharmaceutically acceptable salt thereof, wherein:
A is C₆-C₁₄-aryl;
B is 5- to 14-membered heteroaryl;
L is -O-;
Y¹ is CH or N;
Y² and Y⁴ are both CH;
Y³ is selected from CH, N, C-halogen and C-C₁-C₆-alkyl;
R¹, R², R³ and R⁶ are each hydrogen;
R⁴ is C₁-C₆-alkyl; and
R⁵ is C₁-C₆-alkoxy; or
R⁴ and R⁵, taken together with the atoms to which they are attached, form a 3- to 14-membered heterocycle.

In a particularly preferred embodiment, the present invention provides a compound of formula (I), or a pharmaceutically acceptable salt thereof, wherein:
A is phenyl;
B is pyridyl;
L is -O-;
Y¹ is CH or N;
Y² and Y⁴ are both CH;
Y³ is selected from CH, N, C-F and C-methyl;
R¹, R², R³ and R⁶ are each hydrogen;
R⁴ is methyl; and
R⁵ is methoxy; or
R⁴ and R⁵, taken together with the atoms to which they are attached, form a 5-oxaspiro[2.4]hept-6-ene or a 5-thiaspiro[2.4]hept-6-ene ring.

In one embodiment, the present invention provides a compound of formula (I), or a pharmaceutically acceptable salt thereof, wherein:
A is selected from C₆-C₁₄-aryl and 5- to 14-membered heteroaryl;
R¹ is selected from hydrogen, halogen and C₁-C₆-alkyl; and
R² and R³ are each hydrogen.

In a preferred embodiment, the present invention provides a compound of formula (I), or a pharmaceutically acceptable salt thereof, wherein:
A is C₆-C₁₄-aryl; and
R¹, R² and R³ are each hydrogen.

In a particularly preferred embodiment, the present invention provides a compound of formula (I), or a pharmaceutically acceptable salt thereof, wherein:
A is phenyl; and
R¹, R² and R³ are each hydrogen.

In one embodiment, the present invention provides a compound of formula (I), or a pharmaceutically acceptable salt thereof, wherein:
B is selected from C₆-C₁₄-aryl, 5- to 14-membered heteroaryl and C₃-C₁₀-cycloalkyl;
R⁴ is selected from C₁-C₆-alkyl, halo-C₁-C₆-alkyl and C₁-C₆-alkoxy; and
R⁵ is selected from halogen, cyano, C₁-C₆-alkoxy and halo-C₁-C₆-alkoxy; or
R⁴ and R⁵, taken together with the atoms to which they are attached, form a 3- to 14-membered heterocycle; and
R⁶ is hydrogen or C₁-C₆-alkyl.

In a preferred embodiment, the present invention provides a compound of formula (I), or a pharmaceutically acceptable salt thereof, wherein:
B is 5- to 14-membered heteroaryl;
R⁴ is C₁-C₆-alkyl; and
R⁵ is C₁-C₆-alkoxy; or
R⁴ and R⁵, taken together with the atoms to which they are attached, form a 3- to 14-membered heterocycle; and
R⁶ is hydrogen.

In a particularly preferred embodiment, the present invention provides a compound of formula (I), or a pharmaceutically acceptable salt thereof, wherein:
B is pyridyl;
R⁴ is methyl; and
R⁵ is methoxy; or
R⁴ and R⁵, taken together with the atoms to which they are attached, form a 5-oxaspiro[2.4]hept-6-ene or a 5-thiaspiro[2.4]hept-6-ene ring; and
R⁶ is hydrogen.

In one embodiment, the present invention provides a compound of formula (I), or a pharmaceutically acceptable salt thereof, wherein:
Y¹, Y² and Y⁴ are each independently selected from CH and N; and
Y³ is selected from CH, N, C-halogen, C-C₁-C₆-alkyl and C-halo-C₁-C₆-alkyl.

In a preferred embodiment, the present invention provides a compound of formula (I), or a pharmaceutically acceptable salt thereof, wherein:
Y¹ is CH or N;
Y² and Y⁴ are both CH; and
Y³ is selected from CH, N, C-halogen and C-C₁-C₆-alkyl.

In a particularly preferred embodiment, the present invention provides a compound of formula (I), or a pharmaceutically acceptable salt thereof, wherein:
Y¹ is CH or N;
Y² and Y⁴ are both CH; and
Y³ is selected from CH, N, C-F and C-methyl.

In a preferred embodiment, the present invention provides a compound of formula (I), or a pharmaceutically acceptable salt thereof, selected from:
3-(6-spiro[2H-benzofuran-3,1'-cyclopropane]-4-yloxy-3-pyridyl)-1H-imidazo[4,5-b]pyridin-2-one;
9-(6-spiro[2H-benzofuran-3,1'-cyclopropane]-4-yloxy-3-pyridyl)-7H-purin-8-one;
3-[6-(3-chloro-4-methyl-phenoxy)-3-pyridyl]-1H-imidazo[4,5-b]pyridin-2-one;
3-tert-butyl-4-[[5-(2-oxo-1H-imidazo[4,5-b]pyridin-3-yl)-2-pyridyl]oxy]benzonitrile;
1-(6-spiro[2H-benzofuran-3,1'-cyclopropane]-4-yloxy-3-pyridyl)-3H-imidazo[4,5-c]pyridin-2-one;
6-fluoro-3-[6-(3-methoxy-4-methyl-phenoxy)-3-pyridyl]-1H-imidazo[4,5-b]pyridin-2-one;
3-(3-fluoro-4-spiro[2H-benzofuran-3,1'-cyclopropane]-4-yloxy-phenyl)-1H-imidazo [4,5-b]pyridin-2-one;
1-[6-[4-methyl-3-(trifluoromethoxy)phenoxy]-3-pyridyl]-3H-imidazo[4,5-c]pyridin-2-one;
3-[6-(3-methoxy-4-methyl-phenoxy)-3-pyridyl]-6-methyl-1H-imidazo[4,5-b]pyridin-2-one;
3-(6-spiro[2H-benzofuran-3,1'-cyclopropane]-4-yloxy-3-pyridyl)-1H-benzimidazol-2-one;
2-isopropyl-4-[[5-(2-oxo-1H-imidazo[4,5-b]pyridin-3-yl)-2-pyridyl]oxy]benzonitrile;
4-[[5-(6-fluoro-2-oxo-1H-imidazo[4,5-b]pyridin-3-yl)-2-pyridyl]oxy]-2-isopropyl-benzonitrile;
3-(5-methyl-6-spiro[2H-benzofuran-3,1'-cyclopropane]-4-yloxy-3-pyridyl)-1H-benzimidazol-2-one;
1-[5-fluoro-6-(3-methoxy-4-methyl-phenoxy)-3-pyridyl]-3H-imidazo[4,5-c]pyridin-2-one;
1-[6-(3-methoxy-4-methyl-phenoxy)-5-methyl-3-pyridyl]-3H-imidazo[4,5-c]pyridin-2-one;
3-[6-(3-methoxy-4-methyl-phenoxy)-3-pyridyl]-1H-imidazo[4,5-b]pyridin-2-one;
1-(6-spiro[2H-benzofuran-3,1'-cyclopropane]-4-yloxy-3-pyridyl)-3H-imidazo[4,5-b]pyridin-2-one;
2-isopropyl-4-[[5-(8-oxo-7H-purin-9-yl)-2-pyridyl]oxy]benzonitrile;
3-(3-fluoro-4-spiro[2H-benzofuran-3,1'-cyclopropane]-4-yloxy-phenyl)-1H-benzimidazol-2-one;
1-[3-(3-methoxy-4-methyl-phenoxy)cyclobutyl]-3H-imidazo[4,5-c]pyridin-2-one;
3-[6-(3-fluoro-4-methyl-phenoxy)-3-pyridyl]-1H-imidazo[4,5-b]pyridin-2-one;
3-(6-spiro[2H-benzofuran-3,1'-cyclopropane]-4-yloxy-3-pyridyl)-1H-imidazo[4,5-c]pyridin-2-one;
3-[6-(3-methoxy-4-methyl-phenoxy)-3-pyridyl]-1H-imidazo[4,5-b]pyrazin-2-one;
3-tert-butyl-4-[[5-(8-oxo-7H-purin-9-yl)-2-pyridyl]oxy]benzonitrile;
3-[6-(3-methoxy-4-methyl-phenoxy)-5-methyl-3-pyridyl]-1H-imidazo[4,5-b]pyridin-2-one;
2-ethyl-4-[[5-(2-oxo-1H-imidazo[4,5-b]pyridin-3-yl)-2-pyridyl]oxy]benzonitrile;
3-[6-(2-fluoro-4-methyl-phenoxy)-3-pyridyl]-1H-imidazo[4,5-b]pyridin-2-one;
1-[6-(3-methoxy-4-methyl-phenoxy)-3-pyridyl]-3H-imidazo[4,5-c]pyridin-2-one;
4-[2-fluoro-4-(2-oxo-1H-imidazo[4,5-b]pyridin-3-yl)phenoxy]-2-isopropyl-benzonitrile;
3-tert-butyl-4-[[5-(2-oxo-3H-imidazo[4,5-c]pyridin-1-yl)-2-pyridyl]oxy]benzonitrile;
3-[6-(2-methoxy-4-methyl-phenoxy)-3-pyridyl]-1H-imidazo[4,5-b]pyridin-2-one;
3,5-dimethyl-4-[[5-(2-oxo-1H-imidazo[4,5-b]pyridin-3-yl)-2-pyridyl]oxy]benzonitrile; 3-[6-(3-methoxy-4-methyl-phenoxy)-3-pyridyl]-1H-benzimidazol-2-one;
3-tert-butyl-4-[[5-(6-fluoro-2-oxo-1H-imidazo[4,5-b]pyridin-3-yl)-2-pyridyl]oxy] benzonitrile;
3-[6-(3-methoxy-4-methyl-phenoxy)-3-pyridyl]-6-(trifluoromethyl)-1H-imidazo[4,5-b]pyridin-2-one;
9-[6-[4-methyl-3-(trifluoromethoxy)phenoxy]-3-pyridyl]-7H-purin-8-one;
2-isopropyl-4-[[5-(2-oxo-3H-imidazo[4,5-c]pyridin-1-yl)-2-pyridyl]oxy]benzonitrile;
3-methyl-4-[[5-(2-oxo-1H-imidazo[4,5-b]pyridin-3-yl)-2-pyridyl]oxy]benzonitrile;
4-[[5-(2-oxo-1H-imidazo[4,5-b]pyridin-3-yl)-2-pyridyl]oxy]-3-(trifluoromethyl)benzonitrile;
9-[6-(3-methoxy-4-methyl-phenoxy)-3 -pyridyl]-7H-purin-8-one;
6-chloro-3-[6-(3-methoxy-4-methyl-phenoxy)-3-pyridyl]-1H-imidazo[4,5-b]pyridin-2-one;
1-[6-(3-chloro-4-methyl-phenoxy)-3-pyridyl]-3H-imidazo[4,5-c]pyridin-2-one;
3-tert-butyl-4-[[5-(2-oxo-3H-benzimidazol-1-yl)-2-pyridyl]oxy]benzonitrile;
9-[6-(3-chloro-4-methyl-phenoxy)-3-pyridyl]-7H-purin-8-one;
3-tert-butyl-4-[2-fluoro-4-(2-oxo-1H-imidazo[4,5-b]pyridin-3-yl)phenoxy]benzonitrile;
2-isopropyl-4-[[5-(2-oxo-3H-benzimidazol-1-yl)-2-pyridyl]oxy]benzonitrile;
3-[6-[(5-methoxy-6-methyl-3-pyridyl)oxy]-3-pyridyl]-1H-imidazo[4,5-b]pyridin-2-one;
3-isopropyl-4-[3-(2-oxo-1H-imidazo[4,5-b]pyridin-3-yl)cyclobutoxy]benzonitrile;
2-ethyl-4-[[5-(2-oxo-3H-benzimidazol-1-yl)-2-pyridyl]oxy]benzonitrile;
4-[2-fluoro-4-(8-oxo-7H-purin-9-yl)phenoxy]-2-isopropyl-benzonitrile;
1-[6-(3-methoxy-4-methyl-phenoxy)-3-pyridyl]-3H-imidazo[4,5-b]pyridin-2-one;
2-isopropoxy-4-[[5-(2-oxo-3H-benzimidazol-1-yl)-2-pyridyl]oxy]benzonitrile; and
3-[6-(3-methoxy-4-methyl-phenoxy)-3-pyridyl]-1H-imidazo[4,5-c]pyridin-2-one.

In a particularly preferred embodiment, the present invention provides a compound of formula (I), or a pharmaceutically acceptable salt thereof, selected from:
3-(6-spiro[2H-benzofuran-3,1'-cyclopropane]-4-yloxy-3-pyridyl)-1H-imidazo[4,5-b]pyridin-2-one;
9-(6-spiro[2H-benzofuran-3,1'-cyclopropane]-4-yloxy-3-pyridyl)-7H-purin-8-one;
1-(6-spiro[2H-benzofuran-3,1'-cyclopropane]-4-yloxy-3-pyridyl)-3H-imidazo[4,5-c]pyridin-2-one;
6-fluoro-3-[6-(3-methoxy-4-methyl-phenoxy)-3-pyridyl]-1H-imidazo[4,5-b]pyridin-2-one;
3-[6-(3-methoxy-4-methyl-phenoxy)-3-pyridyl]-6-methyl-1H-imidazo[4,5-b]pyridin-2-one;
3-[6-(3-methoxy-4-methyl-phenoxy)-3-pyridyl]-1H-imidazo[4,5-b]pyridin-2-one; and
1-[6-(3-methoxy-4-methyl-phenoxy)-3-pyridyl]-3H-imidazo[4,5-c]pyridin-2-one.

In one embodiment, the present invention provides pharmaceutically acceptable salts of the compounds according to formula (I) as described herein, especially hydrochloride salts. In a further embodiment, the present invention provides compounds according to formula (I) as described herein as free bases.

In some embodiments, the compounds of formula (I) are isotopically-labeled by having one or more atoms therein replaced by an atom having a different atomic mass or mass number. Such isotopically-labeled (i.e., radiolabeled) compounds of formula (I) are considered to be within the scope of this disclosure. Examples of isotopes that can be incorporated into the compounds of formula (I) include isotopes of hydrogen, carbon, nitrogen, oxygen, phosphorous, sulfur, fluorine, chlorine, and iodine, such as, but not limited to, ²H, ³H, ¹¹C, ¹³C, ¹⁴C, ¹³N, ¹⁵N, ¹⁵O, ¹⁷O, ¹⁸O, ³¹P, ³²P, ³⁵S, ¹⁸F, ³⁶Cl, ¹²³I, and ¹²⁵I, respectively. Certain isotopically-labeled compounds of formula (I), for example, those incorporating a radioactive isotope, are useful in drug and/or substrate tissue distribution studies. The radioactive isotopes tritium, i.e. ³H, and carbon-14, i.e., ¹⁴C, are particularly useful for this purpose in view of their ease of incorporation and ready means of detection. For example, a compound of formula (I) can be enriched with 1, 2, 5, 10, 25, 50, 75, 90, 95, or 99 percent of a given isotope.

Substitution with heavier isotopes such as deuterium, i.e. ²H, may afford certain therapeutic advantages resulting from greater metabolic stability, for example, increased in vivo half-life or reduced dosage requirements.

Substitution with positron emitting isotopes, such as ¹¹C, ¹⁸F, ¹⁵O and ¹³N, can be useful in Positron Emission Topography (PET) studies for examining substrate receptor occupancy. Isotopically-labeled compounds of formula (I) can generally be prepared by conventional techniques known to those skilled in the art or by processes analogous to those described in the Examples as set out below using an appropriate isotopically-labeled reagent in place of the non-labeled reagent previously employed.

### Processes of Manufacturing

The preparation of compounds of formula (I) of the present invention may be carried out in sequential or convergent synthetic routes. Syntheses of the invention are shown in the following general schemes. The skills required for carrying out the reaction and purification of the resulting products are known to those persons skilled in the art. The substituents and indices used in the following description of the processes have the significance given herein, unless indicated to the contrary.

If one of the starting materials, intermediates or compounds of formula (I) contain one or more functional groups which are not stable or are reactive under the reaction conditions of one or more reaction steps, appropriate protective groups (as described e.g., in "Protective Groups in Organic Chemistry" by T. W. Greene and P. G. M. Wutts, 5th Ed., 2014, John Wiley & Sons, N.Y.) can be introduced before the critical step applying methods well known in the art. Such protective groups can be removed at a later stage of the synthesis using standard methods described in the literature.

If starting materials or intermediates contain stereogenic centers, compounds of formula (I) can be obtained as mixtures of diastereomers or enantiomers, which can be separated by methods well known in the art e.g., chiral HPLC, chiral SFC or chiral crystallization. Racemic compounds can e.g., be separated into their antipodes via diastereomeric salts by crystallization with optically pure acids or by separation of the antipodes by specific chromatographic methods using either a chiral adsorbent or a chiral eluent. It is equally possible to separate starting materials and intermediates containing stereogenic centers to afford diastereomerically/enantiomerically enriched starting materials and intermediates. Using such diastereomerically/enantiomerically enriched starting materials and intermediates in the synthesis of compounds of formula (I) will typically lead to the respective diastereomerically/enantiomerically enriched compounds of formula (I).

A person skilled in the art will acknowledge that in the synthesis of compounds of formula (I) - insofar not desired otherwise - an "orthogonal protection group strategy" will be applied, allowing the cleavage of several protective groups one at a time each without affecting other protective groups in the molecule. The principle of orthogonal protection is well known in the art and has also been described in literature (e.g. Barany and R. B. Merrifield, J. Am. Chem. Soc. 1977, 99, 7363; H. Waldmann et al., Angew. Chem. Int. Ed. Engl. 1996, 35, 2056).

A person skilled in the art will acknowledge that the sequence of reactions may be varied depending on reactivity and nature of the intermediates.

In more detail, the compounds of formula (I) can be manufactured by the methods given below, by the methods given in the examples or by analogous methods. Appropriate reaction conditions for the individual reaction steps are known to a person skilled in the art. Also, for reaction conditions described in literature affecting the described reactions see for example:
Comprehensive Organic Transformations: A Guide to Functional Group Preparations, 2nd Edition, Richard C. Larock. John Wiley & Sons, New York, NY. 1999*).* It was found convenient to carry out the reactions in the presence or absence of a solvent. There is no particular restriction on the nature of the solvent to be employed, provided that it has no adverse effect on the reaction or the reagents involved and that it can dissolve the reagents, at least to some extent.

The described reactions can take place over a wide range of temperatures, and the precise reaction temperature is not critical to the invention. It is convenient to carry out the described reactions in a temperature range between -78 °C to reflux. The time required for the reaction may also vary widely, depending on many factors, notably the reaction temperature and the nature of the reagents. However, a period of from 0.5 hours to several days will usually suffice to yield the described intermediates and compounds. The reaction sequence is not limited to the one displayed in the schemes, however, depending on the starting materials and their respective reactivity, the sequence of reaction steps can be freely altered.

If starting materials or intermediates are not commercially available or their synthesis not described in literature, they can be prepared in analogy to existing procedures for close analogues or as outlined in the experimental section.

The present compounds of formula (I) can be prepared by reacting a (hetero)aromatic cis-diamine of formula **1** with a coupling agent (such as CDI, triphosgene), in a solvent (such as DCM, CH₃CN, THF) and often in the presence of a mild base (e.g. TEA, DIEA, pyridine).

### (Scheme 1)

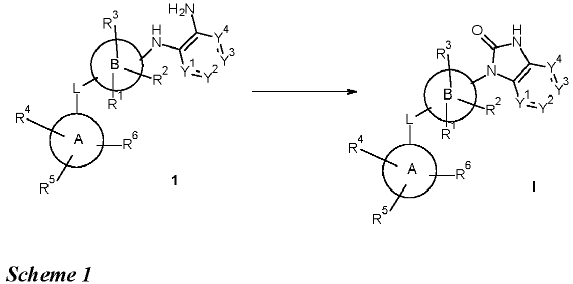

The diamine 1 was typically generated by reduction of a nitro(hetero)aryl derivative 2 (e.g. using Pd/C catalysis under H₂ gas, Fe or Zn in AcOH or EtOH/NH₄Cl (aq.), SnCl₂.2H₂O, or Pd(OH)₂ and triethylsilane). (Scheme 2)

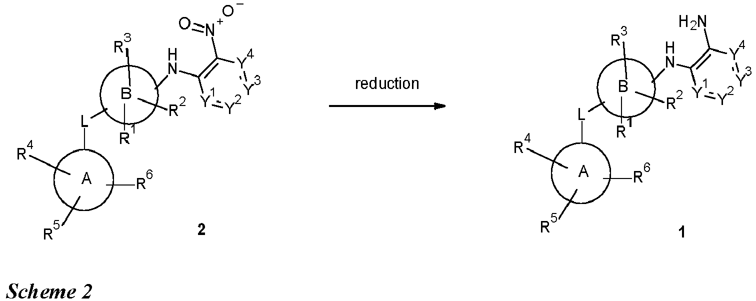

The nitro derivative **2** could be generated by nucleophilic aromatic substitution of an *ortho-halo-*nitro(hetero)aryl derivative with a suitable amine, **3** (e.g. using K₂CO₃ in DMF). Alternatively, for some pyridyl derivatives, citric acid hydrate/dioxane was used. Alternatively the nitro derivative **2** could be generated from the *ortho*-halo-nitro(hetero)aryl derivative and the amine **3** using a transition-metal-catalyzed cross-coupling (e.g. Buchwald reaction using Pd(OAc)₂/Xantphos/K₂CO₃ or or Pd₂(dba)₃/Xantphos/Cs₂CO₃ in 1,4-dioxane). (Scheme 3) Nucleophilic aromatic substitution or metal-catalysed cross-coupling also allows access to the acid/ester and Cl derivatives required for the alternative diamine generation approaches outlined above.

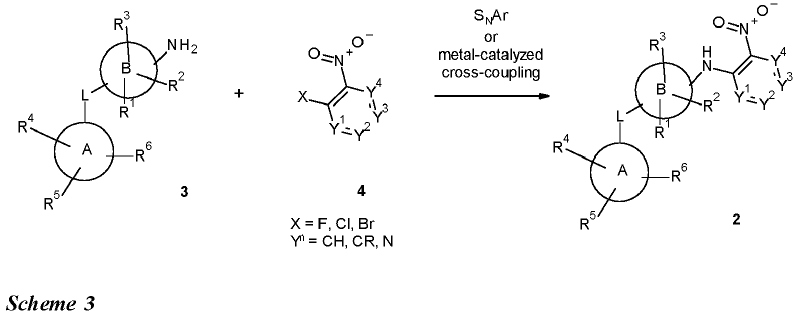

In some cases, the amine, 3, was first protected with a suitable protecting group (e.g. Boc), before the S_{N}Ar and reduction steps (Schemes 2 and 3). The protecting group could then be removed (e.g. with TFA) in a separate step, prior to the coupling step (Scheme 1).

Alternative methods to access the diamine **1** include: (i) S_{N}Ar of amine **3** on e.g. 4-chloro-5-bromopyrimidine (TsOH/NMP), followed by conversion of the bromo derivative to an amine via installation of a protected amino group (Buchwald conditions) and deprotection (see Example 43); (ii) reduction of a tetrazolo-derivative using Fe/EtOH/NH₄Cl (aq.), generated via the chloro derivative (see Example 48); (iii) Curtius rearrangment from a (hetero)aryl acid derivative.

Amines, **3,** where B is an optionally substituted (hetero)aromatic ring can be generated by reduction of a nitro-derivative, 7, (e.g. using Pd/C catalysis under H₂ gas or with ammonium formate/MeOH, Fe or Zn in AcOH or EtOH/NH₄Cl (aq.), or Pd(OH)₂ and triethylsilane). The required nitro-derivative can be generated by a nucleophilic aromatic substitution reaction of a *para-halo* nitro(hetero)arene (**6**) with a suitable alcohol (**5**) (e.g. using NaH, Cs₂CO₃ or K₂CO₃ in a solvent such as DMF or CH₃CN). (Scheme 4).

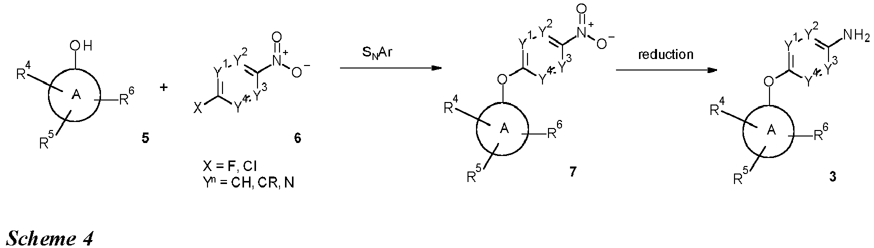

Alternatively, compounds of formula (I) can be prepared by reacting a suitable phenol (**5**) with a suitable 2-chloro-heteroaryl building block (**8**), for example using K₂CO₃ in NMP at 200 °C (Scheme 5).

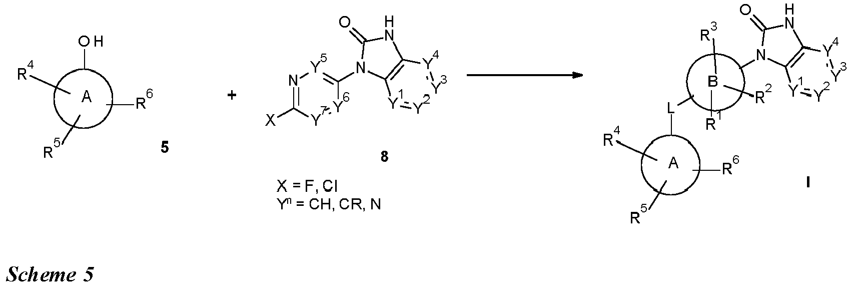

The building block (**8**) could be generated from a 2-chloro-5-amino(hetero)aryl (**9**) using a sequence of S_{N}Ar, reduction of the nitro to the amine, and cyclization of the diamine (in analogy to Schemes 1, 2 and 3). Alternatively, intermediate **10** could be generated by a transition metal-catalyzed cross-coupling of a suitable iodide (**12**) with a suitable amine (**13**) (e.g. Buchwald conditions, Pd₂dba₃/Xantphos/Cs₂CO₃ in dioxane). (Scheme 6).

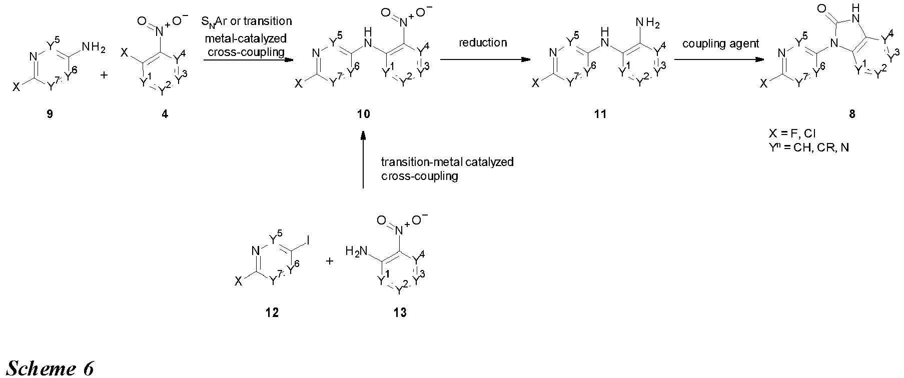

Alternatively, compounds of formula (I) can be prepared from an urea, **15,** using an intramolecular transition-metal catalyzed cross-coupling (e.g. using Pd₂dba₃, dppf and NaO*t*-Bu in dioxane at 100 °C). The urea **15** can be prepared from a suitable amine (**3**) and a suitable *ortho-*halo amino(hetero)arene (**14**) via formation of an actived carbamate (e.g. using 4-nitrophenyl carbonochloridate to install the 4-nitrophenylcarbamate group), followed by coupling with **14** (e.g. using DMAP/THF). (Scheme 7).

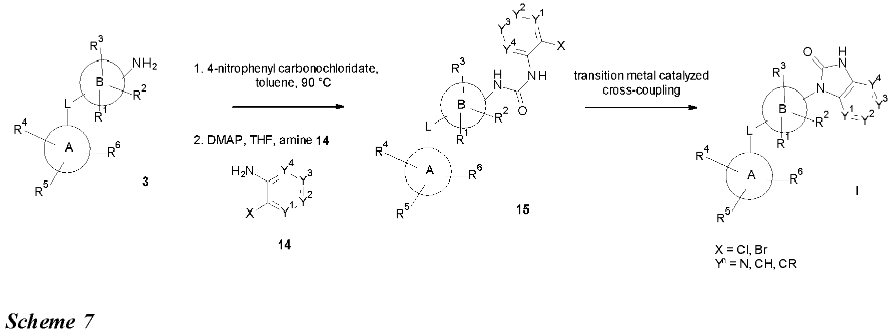

Alternatively, compounds of formula (I) could be generated from a suitable amine, **3,** using transition-metal catalyzed cross-coupling with an *ortho-halo* (hetero)aryl carbamate (**16**) (e.g. Pd(dppf)Cl₂/Xantphos/Cs₂CO₃/1,4-dioxane). (Scheme 8) The required carbamates, **16,** could be synthesized by acylation of a suitable amine with an alkyl chloroformate.

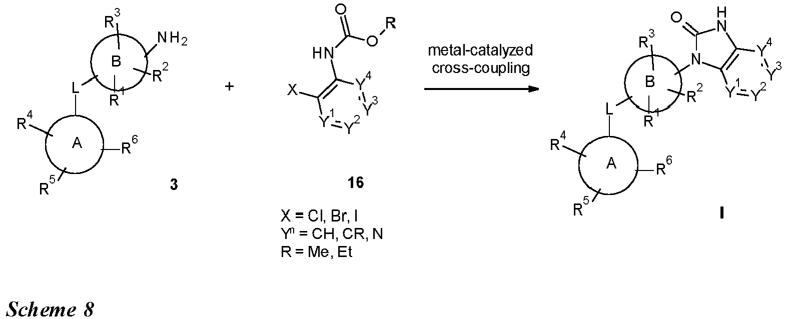

Amines, **3,** where B is a cycloalkyl or heterocyclyl can be generated by reacting a suitable phenol (**5**) with a suitable amino-alcohol (**17**) under Mitsunobu conditions (DIAD, PPh3, CH₂Cl₂) to generate **18,** which could be deprotected under standard conditions to give the amine **3** (e.g. where PG = Boc, deprotection with TFA). (Scheme 9) Compounds of formula (I) could then be generated using the synthetic approaches described previously.

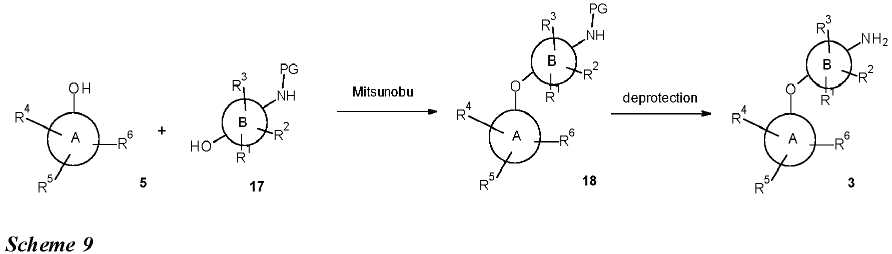

Alternatively, compounds of formula (I), where B is a cycloalkyl or heterocyclyl, can be generated by reacting a cycloalkyl or heterocyclyl-alcohol (**21**) with a suitable aromatic chloride (with para EWG) (**22**) in an S_{N}Ar reaction (e.g. with NaH in DMF). The required alcohol could be generated by the intramolecular transition-metal catalysed cross-coupling of a suitable urea (**20**) (e.g. using Pd₂dba₃/dppf/NaO*t*-Bu/dioxane). The urea could be generated by the coupling of a suitable (hetero)aromatic halide (**19**) with a suitable cycloalkyl or heterocyclyl alcohol (**23**) (e.g. using phenyl chloroformate/DIPEA). (Scheme 10). Non-commercial aromatic chlorides could be generated from the corresponding amines under Sandmeyer conditions (e.g. t-Bu nitrite with CuCl₂).

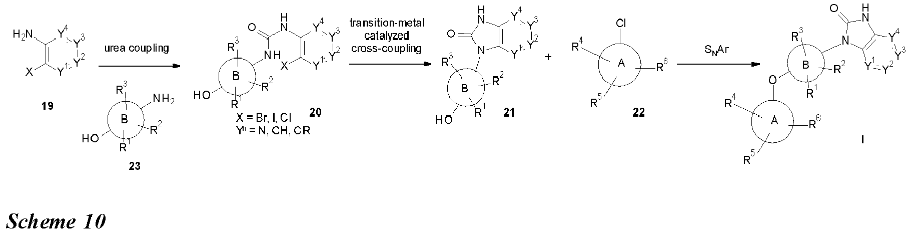

Alternatively, compounds of formula (I), can also be generated by modification of other compounds of formula (I) at a late stage (e.g. where Rₓ = I or Br, installing small alkyl groups via Suzuki cross-coupling with a suitable boronate; see Example 47).

In one aspect, the present invention provides a process of manufacturing the compounds of formula (I) described herein, comprising reacting a diamine of formula **1** 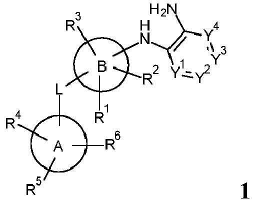 with a coupling agent, such as CDI or triphosgene, to form said compound of formula (I).

In one embodiment, the reaction is performed in a solvent, such as DCM, CH₃CN or THF, or a mixture thereof.

In one embodiment, the reaction is performed in the presence of a mild base, e.g. TEA, DIEA or pyridine, or a mixture thereof.

In one aspect, the present invention provides a compound of formula (I) as described herein, when manufactured according to any one of the processes described herein.

### Using the Compounds of the Invention

Compounds of the present invention are Kv3 enhancers (or positive modulators) that have therapeutic utility. Thus, in one aspect, the present invention provides compounds of formula (I) as described herein for use as therapeutically active substance.

In a further aspect, the present invention provides the use of compounds of formula (I) as described herein for enhancing Kv3 in a mammal.

In a further aspect, the present invention provides compounds of formula (I) as described herein for use in a method of enhancing Kv3 in a mammal.

In a further aspect, the present invention provides the use of compounds of formula (I) as described herein for the preparation of a medicament for enhancing Kv3 in a mammal.

In a further aspect, the present invention provides a method of enhancing Kv3 in a mammal, which method comprises administering an effective amount of a compound of formula (I) as described herein to the mammal.

In a further aspect, the present invention provides a compound of formula (I) as described herein, or a pharmaceutically acceptable salt thereof, or a pharmaceutical composition comprising said compound of formula (I) or a pharmaceutically acceptable salt thereof, for use in the treatment or prophylaxis of Kv3 mediated diseases and disorders.

In a further aspect, the present invention provides the use of a compound of formula (I) as described herein, or a pharmaceutically acceptable salt thereof, or of a pharmaceutical composition comprising said compound of formula (I) or a pharmaceutically acceptable salt thereof, in the treatment or prophylaxis of Kv3 mediated diseases and disorders.

In a further aspect, the present invention provides the use of a compound of formula (I) as described herein, or a pharmaceutically acceptable salt thereof, in the manufacture of a medicament for the treatment or prophylaxis of Kv3 mediated diseases and disorders.

In a further aspect, the present invention provides a method of treatment or prophylaxis of Kv3 mediated diseases and disorders, said method comprising administering a therapeutically effective amount of a compound of formula (I) as described herein, or a pharmaceutically acceptable salt thereof, to a subject in need thereof.

In one embodiment, said Kv3 mediated diseases and disorders are neurodevelopmental disorders.

In one embodiment, said Kv3 mediated diseases and disorders are neurodevelopmental disorders selected from the group consisting of autism, fragile X syndrome, epilepsy, intellectual disability, cognitive impairment, ataxia, depression, schizophrenia, attention deficit hyperactivity disorder and sensory processing disorders (e.g., auditory sensory processing disorders).

In one embodiment, said Kv3 mediated diseases and disorders are autism.

In one embodiment, said Kv3 mediated diseases and disorders are fragile X syndrome.

In one embodiment, said Kv3 mediated diseases and disorders are epilepsy.

In one embodiment, said Kv3 mediated diseases and disorders are intellectual disability.

In one embodiment, said Kv3 mediated diseases and disorders are cognitive impairment.

In one embodiment, said Kv3 mediated diseases and disorders are ataxia.

In one embodiment, said Kv3 mediated diseases and disorders are depression.

In one embodiment, said Kv3 mediated diseases and disorders are schizophrenia.

In one embodiment, said Kv3 mediated diseases and disorders are attention deficit hyperactivity disorder.

In one embodiment, said Kv3 mediated diseases and disorders are sensory processing disorders (e.g. auditory sensory processing disorders).

### Pharmaceutical Compositions and Administration

In one aspect, the present invention provides a pharmaceutical composition comprising a compound of formula (I) as described herein and a therapeutically inert carrier.

In one embodiment, the present invention provides the pharmaceutical compositions disclosed in Examples 55 and 56.

The compounds of formula (I) and their pharmaceutically acceptable salts can be used as medicaments (e.g. in the form of pharmaceutical preparations). The pharmaceutical preparations can be administered internally, such as orally (e.g. in the form of tablets, coated tablets, dragées, hard and soft gelatin capsules, solutions, emulsions or suspensions), nasally (e.g. in the form of nasal sprays) or rectally (e.g. in the form of suppositories). However, the administration can also be effected parentally, such as intramuscularly or intravenously (e.g. in the form of injection solutions).

The compounds of formula (I) and their pharmaceutically acceptable salts can be processed with pharmaceutically inert, inorganic or organic adjuvants for the production of tablets, coated tablets, dragées and hard gelatin capsules. Lactose, corn starch or derivatives thereof, talc, stearic acid or its salts etc. can be used, for example, as such adjuvants for tablets, dragées and hard gelatin capsules.

Suitable adjuvants for soft gelatin capsules are, for example, vegetable oils, waxes, fats, semisolid substances and liquid polyols, etc.

Suitable adjuvants for the production of solutions and syrups are, for example, water, polyols, saccharose, invert sugar, glucose, etc.

Suitable adjuvants for injection solutions are, for example, water, alcohols, polyols, glycerol, vegetable oils, etc.

Suitable adjuvants for suppositories are, for example, natural or hardened oils, waxes, fats, semisolid or liquid polyols, etc.

Moreover, the pharmaceutical preparations can contain preservatives, solubilizers, viscosity-increasing substances, stabilizers, wetting agents, emulsifiers, sweeteners, colorants, flavorants, salts for varying the osmotic pressure, buffers, masking agents or antioxidants. They can also contain still other therapeutically valuable substances.

The dosage can vary in wide limits and will, of course, be fitted to the individual requirements in each particular case. In general, in the case of oral administration a daily dosage of about 0.1 mg to 20 mg per kg body weight, preferably about 0.5 mg to 4 mg per kg body weight (e.g. about 300 mg per person), divided into preferably 1-3 individual doses, which can consist, for example, of the same amounts, should be appropriate. It will, however, be clear that the upper limit given herein can be exceeded when this is shown to be indicated.

### Examples

The invention will be more fully understood by reference to the following examples. The claims should not, however, be construed as limited to the scope of the examples.

In case the preparative examples are obtained as a mixture of enantiomers, the pure enantiomers can be separated by methods described herein or by methods known to the man skilled in the art, such as e.g., chiral chromatography (e.g., chiral SFC) or crystallization.

All reaction examples and intermediates were prepared under an argon atmosphere if not specified otherwise.

### Abbreviations

AcOH = acetic acid, ACN = acetonitrile , Bn = benzyl, BINAP = (2,2'-bis(diphenylphosphino)-1,1'-binaphthyl), Boc = tert-butyloxycarbonyl, CAS RN = chemical abstracts registration number, Cbz = benzyloxycarbonyl, Cs₂CO₃ = cesium carbonate, CO = carbon monoxide, CuCl = copper(I) chloride, CuCN = copper(I) cyanide, CuI = copper(I) iodide, DABCO = 1,4-Diazabicyclo[2.2.2]octane;triethylenediamine, DAST = (diethylamino)sulfur trifluoride, DBU = 1,8-diazabicyclo[5,4,0]undec-7-ene, DEAD = diethyl azodicarboxylate, DIAD = diisopropyl azodicarboxylate, DIBAL-H = diisobutyl aluminium hydride, DMAP = 4-dimethylaminopyridine, DME = dimethoxyethane , DMEDA = N,N'-dimethylethylenediamine, DMF = N,N-dimethylformamide, DIPEA = N,N-diisopropylethylamine, dppf = 1,1 bis(diphenyl phosphino)ferrocene, EDC.HCl = N-(3-dimethylaminopropyl)-N'-ethylcarbodiimide hydrochloride, EI = electron impact, ESI = electrospray ionization, EtOAc = ethyl acetate, EtOH = ethanol, h = hour(s), FA = formic acid, H₂O = water, H₂SO₄ = sulfuric acid, HATU = 1-[bis(dimethylamino)methylene]-1H-1,2,3-triazolo[4,5-b]pyridinium-3-oxide hexafluorophosphate, HBTU = O-benzotriazole-N,N,N',N'-tetramethyl-uronium-hexafluorophosphate, HCl = hydrogen chloride, HOBt = 1-hydroxy-1H-benzotriazole; HPLC = high performance liquid chromatography, iPrMgCl = isopropylmagnesium chloride, 12 = iodine, IPA = 2-propanol, ISP = ion spray positive (mode), ISN = ion spray negative (mode), K₂CO₃ = potassium carbonate, KHCO₃ = potassium bicarbonate, KI = potassium iodide, KOH = potassium hydroxide, K₃PO₄ = potassium phosphate tribasic, LiAlH₄ or LAH = lithium aluminium hydride, LiHMDS = lithium bis(trimethylsilyl)amide, LiOH = lithium hydroxide, mCPBA = meta-chloroperoxybenzoic acid, MgSO₄ = magnesium sulfate, min = minute(s), mL = milliliter, MPLC = medium pressure liquid chromatography, MS = mass spectrum, nBuLi = n-butyllithium, NaBH₃CN = sodium cyanoborohydride, NaH = sodium hydride, NBS = N-bromosuccinimide, NaHCO₃ = sodium hydrogen carbonate, NaNO₂ = sodium nitrite, NaBH(OAc)₃ = sodium triacetoxyborohydride, NaOH = sodium hydroxide, Na₂CO₃ = sodium carbonate, Na₂SO₄ = sodium sulfate, Na₂S₂O₃ = sodium thiosulfate, NEt₃ = triethylamine (TEA), NH₄Cl = ammonium chloride, NMP = N-methyl-2-pyrrolidone, OAc = Acetoxy, T3P = propylphosphonic anhydride, PE = petroleum ether, PG = protective group, Pd-C = palladium on activated carbon, PdCl2(dppf)-CH₂Cl₂ = 1,1'-bis(diphenylphosphino)ferrocene-palladium(II)dichloride dichloromethane complex, Pd₂(dba)₃ = tris(dibenzylideneacetone)dipalladium(0), Pd(OAc)₂ = palladium(II) acetate, Pd(OH)₂ = palladium hydroxide, Pd(PPh₃)₄ = tetrakis(triphenylphosphine)palladium(0), PTSA = p-toluenesulfonic acid, R = any group, RP = reverse phase, RT = room temperature, SFC = Supercritical Fluid Chromatography, S-PHOS = 2-dicyclohexylphosphino-2',6'-dimethoxybiphenyl, TBAI = tetra butyl ammonium iodine, TEA = triethylamine, TFA = trifluoroacetic acid, THF = tetrahydrofuran, TMEDA = N,N,N',N'-tetramethylethylenediamine, ZnCl₂ = zinc chloride, Hal = halogen, prep-TLC = preparative thin layer chromatography

### Example 1

### 3-(6-spiro[2H-benzofuran-3,1'-cyclopropane]-4-yloxy-3-pytidyl)-1H-imidazo[4,5-b]pyridin-2-one

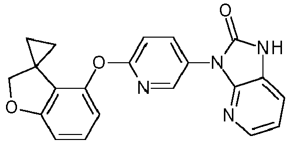

To a solution of N2-(6-spiro[2H-benzofuran-3,1'-cyclopropane]-4-yloxy-3-pyridyl)pyridine-2,3-diamine (0.170 g, 491 µmol) and TEA (149 mg, 1.47 mmol, 205 µL) in DCM (5 mL) cooled to 0 °C was added triphosgene (65.5 mg, 221 µmol) portionwise. The resulting mixture was stirred at 0 °C for 0.5 h. The reaction mixture was poured into water (50 mL), the organic layer was separated, the aqueous phase was extracted with EA (3 x 50 mL), the combined organic layers were dried over anhydrous sulfate sodium, filtered and concentrated. The residue was purified by pre-HPLC to give the title compound (0.116 g, 312 µmol, 63.5 %) as a white solid. MS (ESI): m/z = 373.3 [M+H]⁺

### Step a) 3-nitro-N-(6-spiro[2H-benzofuran-3,1'-cyclopropane]-4-yloxy-3-pyridyl)pyridin-2-amine

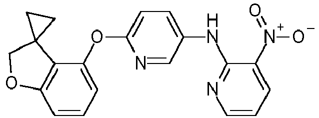

To a solution of compound 2-chloro-3-nitropyridine (0.1 g, 631 µmol) **Example A.1** (160 mg, 631 µmol) Xantphos (73.0 mg, 126 µmol) and K₂CO₃ (131 mg, 946 µmol) in dioxane (3 mL) under nitrogen atmosphere was added Pd(OAc)₂ (14.2 mg, 63.1 µmol). The resulting mixture was stirred at 100 °C for 6 h. The reaction mixture was combined with a replicate, filtered through a pad of celite, rinsed with EA (10 mL x 4), the filtrate was concentrated. The residue was purified by silica gel chromatography (PE:EA = 3:1) to give compound 24-2 (0.25 g, crude) as a light yellow oil which was carried directly to the next step without further purification.

### Step b) N2-(6-spiro[2H-benzofuran-3,1'-cyclopropane]-4-yloxy-3-pyridyl)pyridine-2,3-diamine

To a solution of compound 3-nitro-N-(6-spiro[2H-benzofuran-3,1'-cyclopropane]-4-yloxy-3-pyridyl)pyridin-2-amine (0.5 g, 1.33 mmol) in methanol (25 mL) was added Pd/C (0.2 g, 1.33 mmol, 10% purity), the the reaction mixture was stirred at 50 °C for 1 h under H₂ (30 psi). The reaction mixture was filtered through a pad of celite, the filtrate was concentrated to give N2-(6-spiro[2H-benzofuran-3,1'-cyclopropane]-4-yloxy-3-pyridyl)pyridine-2,3-diamine (0.17 g, 475 µmol, 35.7%, 96.7%) as a off-white solid which was carried into the next step without further purification. MS (ESI): m/z = 347.2 [M+H]⁺

In analogy to Example 1, Examples 10 of the following table were generated, using the respective building blocks in place of 2-chloro-3-nitropyridine in Step a)

| **Ex.** | **Structure** | **Systematic Name** | **Building Blocks** | **MS, ESI: m/z** |
|---|---|---|---|---|
| 10 | 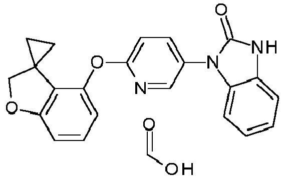 | 3-(6-spiro[2H-benzofuran-3,1'-cyclopropane]-4-yloxy-3-pyridyl)-1H-benzimidazol- 2-one; formic acid salt | 1-fluoro-2-nitrobenzene | 372.1 [M+H]⁺ |

### Example 2

### 9-(6-spiro[2H-benzofuran-3,1'-cyclopropane]-4-yloxy-3-pyridyl)-7H-purin-8-one

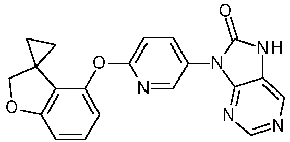

To a solution of 1-(6-((2H-spiro[benzofuran-3,1'-cyclopropan]-4-yl)oxy)pyridin-3-yl)-3-(4-bromopyrimidin-5-yl)urea (300 mg, 660 µmol) in dry dioxane (8 mL) under an inert atmosphere was added Pd₂(dba)₃ (30.2 mg, 33 µmol), 1,1'-ferrocenediyl-bis(diphenylphosphine) (36.6 mg, 66 µmol) and sodium tert-butoxide (95.2 mg, 991 µmol). The reaction mixture was stirred at RT for 5 min and at 100°C for 1.5 h. The reaction mixture was cooled down to RT, quenched by addition of few drops of sat. aq. NH₄Cl solution. The reaction mixture was stirred at RT for 5 min and diluted with ethyl acetate. Insolubles were removed by filtration over a pad of celite, the filter pad was washed with ethyl acetate twice and the filtrate was evaporated down to dryness. The crude material was directly submitted for SFC purification to yield 220 mg of the title compound, which was further purified by flash chromatography eluting with amixture of heptane and a solution of EtOAc:EtOH 3:1 (5% to 70%) to yield 117 mg of the title compound. MS (ESI): m/z = 374.2 [M+H]⁺

### Step a) 4-nitrophenyl (6-((2H-spiro[benzofuran-3,1'-cyclopropan]-4-yl)oxy)pyridin-3-yl)carbamate hydrochloride

To a solution of 6-((2H-spiro[benzofuran-3,1'-cyclopropan]-4-yl)oxy)pyridin-3-amine **Example A.1** (785 mg, 2.93 mmol) in toluene (25 mL) was added 4-nitrophenyl carbonochloridate (591 mg, 2.93 mmol) and the reaction mixture was stirred at 90 °C for 6 h. Volatiles were removed in vacuo to yield 1.45 g of the crude product which was used in the next step without further purification. MS (ESI): m/z = 420.3 [M+H]⁺

### Step b) 1-(6-((2H-spiro[benzofuran-3,1'-cyclopropan]-4-yl)oxy)pyridin-3-yl)-3-(4-bromopyrimidin-5-yl)urea

To a suspension of 4-nitrophenyl (6-((2H-spiro[benzofuran-3,1'-cyclopropan]-4-yl)oxy)pyridin-3-yl)carbamate hydrochloride (1.45 g, 3.02 mmol) in dry THF (20 mL) was added 4-bromopyrimidin-5-amine (631 mg, 3.63 mmol) and DMAP (406 mg, 3.32 mmol). The reaction mixture was then stirred at 70 °C for 4 h.Volatiles were removed in vacuo and the crude material was purified by flash chromatography eluting with an eluent mixture of heptane and a solution EtOAc:EtOH 3:1 (5% to 70%). This yielded a product that was further purified by SFC to yield the title compound (305 mg). MS (ESI): m/z = 456.2 [M+H]⁺

In analogy to Example 2, Examples 3, 7, 9, 28, 29, 50 of the following table were generated from a phenol (Step a) and an amino-halo-heteroarene (Step b)

| **Ex.** | **Structure** | **Systematic Name** | **Building Blocks** | **MS, ESI: m/z** |
|---|---|---|---|---|
| 3 | 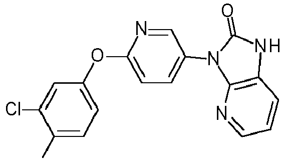 | 3-[6-(3-chloro-4-methyl-phenoxy)-3-pyridyl]-1H-imidazo[4,5-b]pyridin-2-one | A.2 and 2-chloropyridin-3-amine | 353.3 [M+H]⁺ |
| 7 | 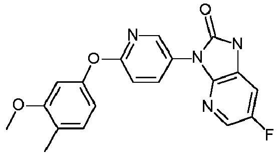 | 6-fluoro-3-[6-(3-methoxy-4-methyl-phenoxy)-3-pyridyl]-1H-imidazo[4,5-b]pyridin-2-one | A.3 and (3-Amino-2-bromo-5-fluoropyridine) CAS: 884495-03-8 | 367.3 [M+H]⁺ |
| 9 | 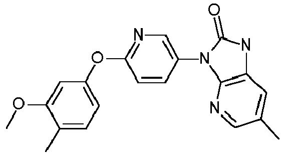 | 3-[6-(3-methoxy-4-methyl-phenoxy)-3-pyridyl]-6-methyl-1H-imidazo[4,5-b]pyridin-2-one | A.3 and 2-chloro-5-methylpyridin-3-amine (CAS: 34552-13-1) | 363.4 [M+H]⁺ |
| 28 | 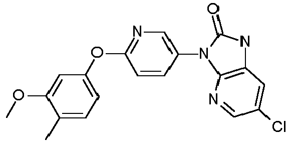 | 6-chloro-3-[6-(3-methoxy-4-methyl-phenoxy)-3-pyridyl]-1H-imidazo[4,5-b]pyridin-2-one | A.3 and 3-amino-2-bromo-5-chloropyridine (CAS: 90902-83-3) | 383.3 [M+H]⁺ |
| 29 | 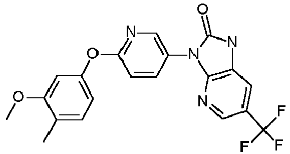 | 3-[6-(3-methoxy-4-methyl-phenoxy)-3-pyridyl]-6-(trifluoromethyl)-1H-imidazo[4,5-b]pyridin-2-one | A.3 and 2-chloro-5-(trifluoromethy l)pyridin-3-amine (CAS: 72587-18-9) | 417.4 [M+H]⁺ |
| 50 | 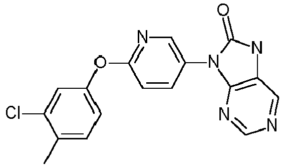 | 9-[6-(3-chloro-4-methyl-phenoxy)-3-pyridyl]-7H-purin-8-one | A.2 and 4-bromopyrimidi n-5-amine | 354.2 [M+H]⁺ |

### Example 4

### 3-tert-butyl-4-[[5-(2-oxo-1H-imidazo[4,5-b]pyridin-3-yl)-2-pyridyl]oxy]benzonitrile

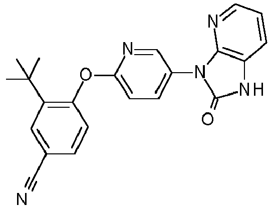

To a solution of 3-(1,1-dimethylethyl)-4-hydroxybenzonitrile (CAS: 4910-04-7) (280 mg, 1.60 mmol) and 3-(6-chloro-3-pyridyl)-1H-imidazo[4,5-b]pyridin-2-one (394 mg, 1.60 mmol) in NMP (3 mL) was added K₂CO₃ (552 mg, 3.99 mmol), and the mixture was stirred at 200 °C for 5 h. The mixture was diluted with water (20 mL) and extracted with ethyl acetate (30 mL x 3), the combined organic phase was dried over sodium sulfate, filtered and concentrated under vacuum to give crude product which was purified by prep-HPLC and lyophilized to give the title compound (22.8 mg, 58.33 µmol, 3.65% yield) as off-white solid. MS (ESI): m/z = 386.4 [M+H]⁺

### Step a) N-(6-chloro-3-pyridyl)-3-nitro-pyridin-2-amine

A mixture of compound 2-chloro-5-iodopyridine (9.64 g, 38.2 mmol), 2-amino-3-nitropyridine (5.32 g, 38.2 mmol), Cs₂CO₃ (18.7 g, 57.4 mmol), Xantphos (4.43 g, 7.65 mmol) and Pd₂(dba)₃ (3.50 g, 3.82 mmol) in dioxane (100 mL) was degassed and purged with N₂ 3 times, and then the mixture was stirred at 80 °C for 2 h under N₂ atmosphere. The reaction mixture was filtered to give a crude product which was purified by flash silica gel chromatography eluting with a 0-20 % ethylacetate/petroleum ether gradient to give the title compound (5.40 g, 20.0 mmol, 52.4% yield) as a yellow solid. MS (ESI): m/z = 451.0 [M+H]⁺

### Step b) N2-(6-chloro-3-pyridyl)pyridine-2, 3-diamine

To a solution ofN-(6-chloro-3-pyridyl)-3-nitro-pyridin-2-amine (4.80 g, 17.2 mmol) in EtOH (50 mL) was added a solution of NH₄Cl (1.38 g, 25.8 mmol) in H₂O (50 mL) .The mixture was heated at 80°C, and then Fe (3.85 g, 68.9 mmol) was added with stirring. The mixture was stirred at 80 °C for 3 h. The reaction mixture was filtered and the filtrate was concentrated under reduced pressure to give a residue which was dissolved with H₂O (100 mL), and the mixture was extracted with ethyl acetate (100 mL x 3). The combined organic phase was washed with brine (200 mL), dried with anhydrous Na₂SO₄, filtered and concentrated in vacuum to give the title compound (3.81 g, 14.9 mmol, 86.6% yield) as a brown solid. MS (ESI): m/z = 221.1 [M+H]⁺

### Step c) 3-(6-chloro-3-pyridyl)-1H-imidazo[4,5-b]pyridin-2-one

To a solution ofN2-(6-chloro-3-pyridyl)pyridine-2,3-diamine (3.60 g, 16.3 mmol) in MeCN (30.0 mL) was added CDI (5.29 g, 32.6 mmol), the formed mixture was stirred at 20 °C for 2 h. The mixture was diluted with water (100 mL), and extracted with ethylacetate (100 mL x 2). The organic phase was washed with brine (20 mL x 2), dried over sodium sulfate, filtered and concentrated under vacuum to give the title compound (4.90 g, crude) as a yellow solid, which was used directly without further purification. MS (ESI): m/z = 247.4 [M+H]⁺

In analogy to Example 4, Examples 5, 35, 36, 37, 49 of the following table were generated from intermediate 3-(6-chloro-3-pyridyl)-1H-imidazo[4,5-b]pyridin-2-one using varied phenol building blocks in the final step. In some cases heating was carried out under microwave conditions.

| **Ex.** | **Structure** | **Systematic Name** | **Building Blocks** | **MS, ESI: m/z** |
|---|---|---|---|---|
| 5 | 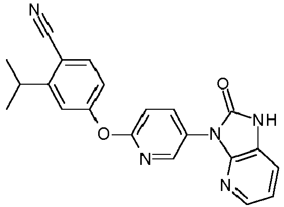 | 2-isopropyl-4-[[5-(2-oxo-1H-imidazo[4,5-b]pyridin-3-yl)-2-pyridyl] oxy] benzonitrile | CAS: 14114-32-0 | 372.4 [M+H]⁺ |
| 35 | 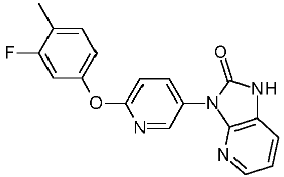 | 3-[6-(3-fluoro-4-methyl-phenoxy)-3 -pyridyl] -1H-imidazo [4,5 -b]pyridin-2-one | 3-fluoro-4-methylphe nol; CAS: 452-78-8 | 337.0 [M+H]⁺ |
| 36 | 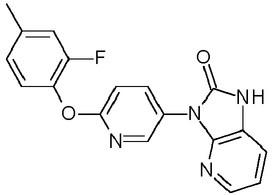 | 3-[6-(2-fluoro-4-methyl-phenoxy)-3 -pyridyl] -1H-imidazo [4,5 -b]pyridin-2-one | 2-fluoro-4-methylphe nol; CAS: 452-81-3 | 337.1 [M+H]⁺ |
| 37 | 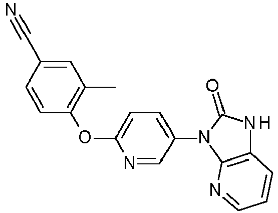 | 3-methyl-4-[[5-(2-oxo-1H-imidazo [4,5 -b]pyridin-3 - yl)-2-pyridyl] oxy] benzonitrile | 4-hydroxy-3-methylben zonitrile; CAS: 15777-70-5 | 344.1 [M+H]⁺ |
| 49 | 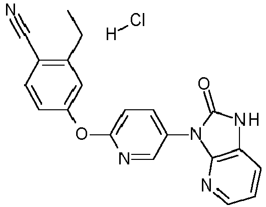 | 2-ethyl-4-[[5-(2-oxo-1H-imidazo [4,5 -b]pyridin-3 - yl)-2-pyridyl] oxy] benzonitrile; hydrochloride salt | 2-ethyl-4-hydroxybe nzonitrile; (CAS: 14143-33-0) | 357.9 [M+H]⁺ |

### Example 6

### 1-(6-spiro[2H-benzofuran-3,1'-cyclopropane]-4-yloxy-3-pyridyl)-3H-imidazo[4,5-c]pyridin-2-one; hydrochloride salt

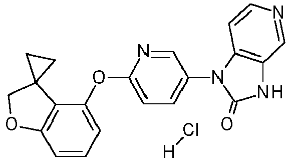

To a mixture of N4-(6-spiro[2H-benzofuran-3,1'-cyclopropane]-4-yloxy-3-pyridyl)pyridine-3,4-diamine (230 mg, 664 µmol) in MeCN (3 mL) was added CDI (215 mg, 1.33 mmol) in one portion. The mixture was stirred at 60 °C for 4 h. The mixture was purified by pre-HPLC and lyophilized to give the title compound (105 mg, 255 µmol, 38.4% yield) as an off-white solid. MS (ESI): m/z = 373.0 [M+H]⁺

### Step a) N-(3-nitro-4-pyridyl)-6-spiro[2H-benzofuran-3,1'-cyclopropane]-4-yloxy-pyridin-3-amine

The mixture of **Example A.1** (150 mg, 590 µmol), 4-chloro-3-nitropyridine (93.5 mg, 590 µmol) and K₂CO₃ (122 mg, 885 µmol) in DMF (2.00 mL) was stirred at 100 °C for 1.5 h. The mixture was diluted with water (60 mL) and extracted with ethyl acetate (80 mL x 3), the organic phase was washed with brine (10 mL x 3) and dried over sodium sulfate, filtered and concentrated under vacuum to give the title compound (285 mg, crude) as a yellow oil which was used directly without further purification. MS (ESI): m/z = 377.3 [M+H]⁺

### Step b) N4-(6-spiro[2H-benzofuran-3,1'-cyclopropane]-4-yloxy-3-pyridyl)pyridine-3,4-diamine

To a solution of N-(3-nitro-4-pyridyl)-6-spiro[2H-benzofuran-3,1'-cyclopropane]-4-yloxy-pyridin-3-amine (285 mg, 757 µmol) in THF (5 mL) was added Pd/C (0.03 g, 10 % purity) under N₂. The suspension was degassed under vacuum and purged with H₂ three times. The mixture was stirred under H₂ (20psi) at 20 °C for 8 h. The mixture was filtered to remove the Pd/C, the filter pad washed with THF (500 mL) and condensed under vacuum to give the title compound (230 mg, crude) as a yellow solid which was used directly without purification.

In analogy to Example 6, Examples 8, 11, 17, 23, 44, 45, 46, 51 were generated from the amine building block A.X and an *ortho* or *para*-halo nitro(hetero)aryl, using a sequence of nucleophilic aromatic substitution, reduction of the nitro group the the amine, and condensation of the diamine with CDI. (*DIEA used as base in Step a) for Examples 44, 45 and 46)

| **Ex.** | **Structure** | **Systematic Name** | **Building Blocks** | **MS, ESI: m/z** |
|---|---|---|---|---|
| 8 | 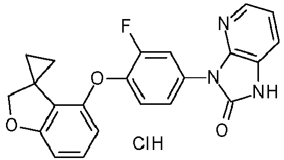 | 3-(3-fluoro-4-spiro[2H-benzofuran-3,1'-cyclopropane]-4-yloxy-phenyl)-1H-imidazo[4,5-b]pyridin-2-one; hydrochloride salt | A.4 and 2-fluoro-3-nitropyridi ne (CAS: 1480-87-1) | 394.6 [M+H]⁺ |
| 11 | 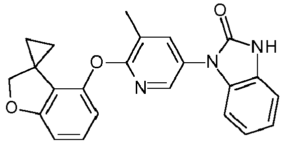 | 3-(5-methyl-6-spiro[2H-benzofuran-3,1'-cyclopropane]-4-yloxy-3-pyridyl)-1H-benzimidazol-2-one | A.5 and 1-fluoro-2-nitrobenze ne | 386.1 [M+H]⁺ |
| 17 | 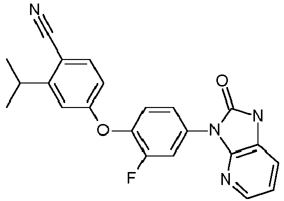 | 4-[2-fluoro-4-(2-oxo-1H-imidazo[4,5-b]pyridin-3-yl)phenoxy]-2-isopropyl-benzonitrile | A.6 and 2-fluoro-3-nitropyridi ne (CAS: 1480-87-1) | 389.3 [M+H]⁺ |
| 23 | 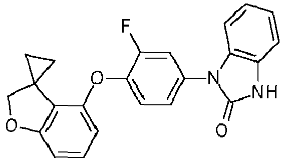 | 3-(3-fluoro-4-spiro[2H-benzofuran-3,1'-cyclopropane]-4-yloxy-phenyl)-lH-benzimidazol-2-one | A.4 and 1-fluoro-2-nitrobenze ne | 388.9 [M+H]⁺ |
| 44 | 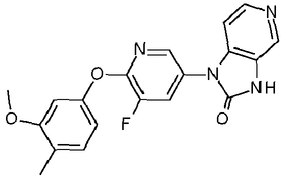 | 1-[5-fluoro-6-(3-methoxy-4-methyl-phenoxy)-3-pyridyl]-3H-imidazo[4,5-c]pyridin-2-one | A.10 and 4-Chloro-3-nitropyridi ne | 367.1 [M+H]⁺ |
| 45 | 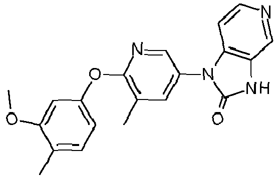 | 1-[5-methyl-6-(3-methoxy-4-methyl-phenoxy)- 3-pyridyl]-3H-imidazo [4,5 -c]pyridin-2-one | A. 11 and 4-Chloro-3-nitropyridi ne | 363.1 [M+H]⁺ |
| 46 | 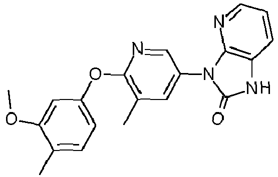 | 3-[6-(3-methoxy-4-methyl-phenoxy)-5-methyl-3-pyridyl]-1H-imidazo[4,5-b]pyridin-2-one | A. 11 and 2-fluoro-3-nitropyridi ne (CAS: 1480-87-1) | 363.1 [M+H]⁺ |
| 51 | 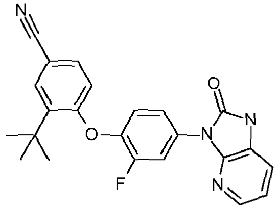 | 3-tert-butyl-4-[2-fluoro-4-(2-oxo-1H-imidazo[4,5-b]pyridin-3-yl)phenoxy]benzonitrile | A.12 and 2-fluoro-3-nitropyridi ne (CAS: 1480-87-1) (CH₂OH)₂ as solvent in Step a) | 403.0 [M+H]⁺ |

### Example 12

### 3-(6-spiro[2H-benzofuran-3,1'-cyclopropane]-4-yloxy-3-pyridyl)-1H-imidazo[4,5-c]pyridin-2-one; formic acid salt

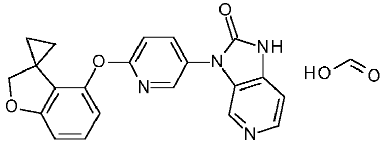

To a solution of N3-(6-spiro[2H-benzofuran-3,1'-cyclopropane]-4-yloxy-3-pyridyl)pyridine-3,4-diamine (140 mg, 0.400 mmol) in THF (5 mL) was added N.N'-carbonyldiimidazole (262 mg, 1.62 mmol). The mixture was stirred at 60 °C for 5 h. The mixture was concentrated to give the residue, which was purified by Prep-HPLC (FA) and lyophilized to give the title compound (106 mg, 0.250 mmol, 62.7% yield) as a white solid. MS (ESI): m/z = 373.1 [M+H]⁺

### Step a) 2-nitro-N-(6-spiro[2H-benzofuran-3,1'-cyclopropane]-4-yloxy-3-pyridyl)pyridin-3-amine

To a mixture of 6-spiro[2H-benzofuran-3,1'-cyclopropane]-4-yloxypyridin-3-amine (Example **A.1**) (150 mg, 0.590 mmol) and 3-bromo-4-nitropyridine (144 mg, 0.710 mmol) in 1,4-Dioxane (15 mL) was added Xantphos (68.3 mg, 0.120 mmo), palladium diacetate (13.2 mg, 0.06 mmol) and potassium carbonate (163 mg, 1.18 mmol) at 25 °C. Then the mixture was stirred at 80 °C for 16 h. The mixture was filtered and concentrated in vacuo to give a crude product, which was purified by column (PE:EtOAc (including 30% EtOH)=20:1 to 3:1) to give N-(4-nitro-3-pyridyl)-6-spiro[2H-benzofuran-3,1'-cyclopropane]-4-yloxy-pyridin-3-amine (210 mg, 0.560 mmol, 94.59% yield) as a yellow solid. MS (ESI): m/z = 377.4 [M+H]⁺

### Step a) N3-(6-spiro[2H-benzofuran-3,1'-cyclopropane]-4-yloxy-3-pyridyl)pyridine-3,4-diamine

To a solution of 2-nitro-N-(6-spiro[2H-benzofuran-3,1'-cyclopropane]-4-yloxy-3-pyridyl)pyridin-3-amine (220 mg, 0.580 mmol) in ethanol (4 mL)/water (1 mL) was added Zn powder (150 mg, 2.34 mmol) and ammonium chloride (124 mg, 2.34 mmol). The mixture was stirred at 25 °C for 2 h.. To the mixture was added Na₂SO₄ and the mixture stirred for 5 min. The mixture was filtered and the filtrate was concentrated to give crude N3-(6-spiro[2H-benzofuran-3,1'-cyclopropane]-4-yloxy-3-pyridyl)pyridine-2,3-diamine (237 mg, 0.680 mmol, 117 % yield) as a yellow solid. MS (ESI): m/z = 347.5 [M+H]⁺

In analogy to Example 12, Examples 13, 14, 27 of the following table were generated from the amine building block A.X and an *ortho* or *para*-halo nitro(hetero)aryl.

| **Ex.** | **Structure** | **Systematic Name** | **Building Blocks** | **MS, ESI: m/z** |
|---|---|---|---|---|
| 13 | 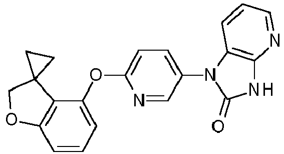 | 1-(6-spiro[2H-benzofuran-3,1'-cyclopropane]-4-yloxy-3-pyridyl)-3H-imidazo [4,5 -b]pyridin-2-one | A.1 and 3-bromo-2-nitropyridine | 373.3 [M+H]⁺ |
| 14 | 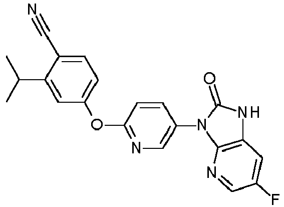 | 4-[[5-(6-fluoro-2-oxo-1H-imidazo[4,5-b]pyridin-3-yl)-2-pyridyl]oxy]-2-isopropyl-benzonitrile | A.7 and 2-bromo-5-fluoro-3-nitropyridine (CAS: 652160-72-0) | 394.2 [M+H]⁺ |
| 27 | 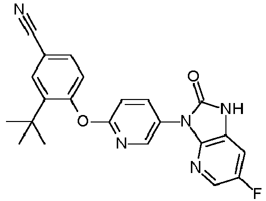 | 3-tert-butyl-4-[[5-(6-fluoro-2-oxo-1H-imidazo[4,5-b]pyridin-3-yl)-2-pyridyl]oxy]benzonitrile | A.8 and 2-bromo-5-fluoro-3-nitropyridine | 404.4 [M+H]⁺ |

### Example 15

### 2-isopropyl-4-[[5-(8-oxo-7H-purin-9-yl)-2-pyridyl]oxy]benzonitrile

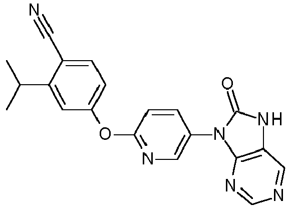

To a mixture of 4-[(5-amino-2-pyridyl)oxy]-2-isopropyl-benzonitrile (Example A.7) (75.4 mg, 0.30 mmol) and ethyl N-(4-chloropyrimidin-5-yl)carbamate (60 mg, 0.30 mmol) in 1,4-Dioxane (4 mL) was added Xantphos (17.2 mg, 0.030 mmol), 1,1'-bis(diphenylphosphino)ferrocene-palladium(II)dichloride dichloromethane complex (12.1 mg, 0.010 mmol) and cesium carbonate (194 mg, 0.600 mmol) at 25 °C. Then the mixture was stirred at 90 °C for 16 h. The mixture was filtered and the filtrate was concentrated to give the residue, which was purified by Prep-HPLC (FA) and lyophilized to give 2-isopropyl-4-[[5-(8-oxo-7H-purin-9-yl)-2-pyridyl]oxy]benzonitrile (43.6 mg, 0.120 mmol, 37.4% yield) as a light yellow solid. MS (ESI): m/z = 373.1 [M+H]⁺

### Step a) Ethyl N-(4-chloropyrimidin-5-yl) carbamate

To a solution of 4-chloropyrimidin-5-amine (1000 mg, 7.72 mmol) in Pyridine (10 mL) was added ethyl chloroformate (1.89 mL, 21.2 mmol). The mixture was stirred at 0 °C for 2 h. The solvent was removed to give the residue, which was purified by flash chromatography (petroleum ether/ethyl acetate=20:1 to 3:1) and concentrated to give ethyl N-(4-chloropyrimidin-5-yl)carbamate (80 mg, 0.400 mmol, 5.1% yield) as a white solid. MS (ESI): m/z = 202.1 [M+H]⁺

In analogy to Example 15, Examples 18, 24, 25 were generated from the amine building block A.X and the relevant ethyl carbamate.

| **Ex.** | **Structure** | **Systematic Name** | **Building Blocks** | **MS, ESI: m/z** |
|---|---|---|---|---|
| 18 | 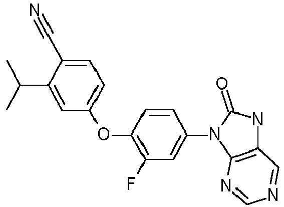 | 4-[2-fluoro-4-(8-oxo-7H-purin-9-yl)phenoxy]-2-isopropyl- benzonitrile | **A.6** and tert-butyl N-(4-chloropyri midin-5-yl)carbam ate (**C.3**) | 389.9 [M+H]⁺ |
| 24 | 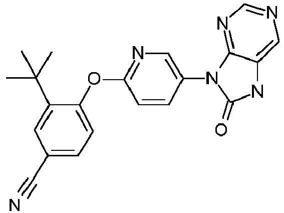 | 3-tert-butyl-4-[[5-(8-oxo-7H-purin-9-yl)-2-pyridyl] oxy] benzonitrile | **A.8** and methyl N-(4-chloropyri midin-5-yl)carbam ate (**C.1**) | 387.3 [M+H]⁺ |
| 25 | 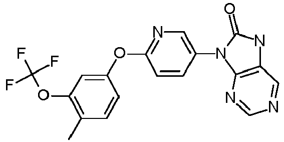 | 9-[6-[4-methyl-3-(trifluoromethoxy)phenox y]-3-pyridyl]-7H-purin-8-one | **A.9** and methyl N-(4-chloropyri midin-5-yl)carbam ate (**C.1**) | 404.0 [M+H]⁺ |

### Example 16

### 2-isopropyl-4-[[5-(2-oxo-3H-imidazo[4,5-c]pyridin-1-yl)-2-pyridyl]oxy]benzonitrile

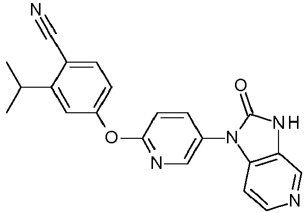

To a solution of 4-hydroxy-2-isopropyl-benzonitrile (CAS: 14114-32-0) (130 mg, 0.810 mmol) in NMP (4 mL) was added 1-(6-chloro-3-pyridyl)-3H-imidazo[4,5-c]pyridin-2-one (200 mg, 0.810 mmol) and potassium carbonate (168 mg, 1.22 mmol) . The mixture was stirred at 200°C for 4 h in a sealed tube under microwave. The mixture was filtered and the filtrate was purified by Prep-HPLC (FA) and lyophilized to give2-isopropyl-4-[[5-(2-oxo-3H-imidazo[4,5-c]pyridin-1-yl)-2-pyridyl]oxy]benzonitrile (8 mg, 0.020 mmol, 2.7 % yield) as grey solid. MS (ESI): m/z = 372.3 [M+H]⁺

### Step a) 6-chloro-N-(3-nitro-4-pyridyl)pyridin-3-amine

To a solution of 2-chloro-5-iodopyridine (10 g, 41.8 mmol) in 1,4-Dioxane (100 mL) was added 4-amino-3-nitropyridine (5.81 g, 41.8 mmol), tris(dibenzylideneacetone)dipalladium (0) (1.91 g, 2.09 mmol) and Xantphos (2.42 g, 4.18 mmol) and cesium carbonate (20.4 g, 62.6 mmol). The mixture was stirred at 90 °C for 4 h. The mixture was filtered and the filtrate was concentrated to give the residue, which was purified by column chromatography on silica (eluting with petroleum ether/ethyl acetate=10:1 to 0:1) and concentrated to give 6-chloro-N-(3-nitro-4-pyridyl)pyridin-3-amine (9.2 g, 36.7 mmol, 87.9 % yield) as a yellow solid. MS (ESI): m/z = 251.5 [M+H]⁺

### Step b) N4-(6-chloro-3-pyridyl)pyridine-3,4-diamine

To a solution of 6-chloro-N-(3-nitro-4-pyridyl)pyridin-3-amine (300 mg, 1.2 mmol) in ethanol (6 mL)/water (2 mL) was added iron (668 mg, 12.0 mmol) and ammonium chloride (323 mg, 5.98 mmol). The mixture was stirred at 70 °C for 4 h. The mixure was filtered and the solid was washed by EtOH for three times. The combined organic layers were concentrated to give N4-(6-chloro-3-pyridyl)pyridine-3,4-diamine (400 mg, 1.81 mmol, 151 % crude yield) as a yellow solid. MS (ESI): m/z = 220.9 [M+H]⁺

### Step c) 1-(6-chloro-3-pyridyl)-3H-imidazo[4,5-c]pyridin-2-one

To a solution ofN4-(6-chloro-3-pyridyl)pyridine-3,4-diamine (100 mg, 0.450 mmol) in MeCN (2 mL) was added N.N'-carbonyldiimidazole (147 mg, 0.910 mmol). The mixture was stirred at 60 °C for 3 h. The mixture was concentrated to give the residue. To the mixture was added MeOH and the mixture was filtered. The filtrate was purified by Prep-HPLC (FA) to give 1-(6-chloro-3-pyridyl)-3H-imidazo[4,5-c]pyridin-2-one (20 mg, 0.080 mmol, 17.9% yield) as light grey solid. MS (ESI): m/z = 247.0 [M+H]⁺

In analogy to Example 16, Examples 26 were generated using the respective phenol building blocks in the final step.

| **Ex.** | **Structure** | **Systematic Name** | **Building Blocks** | **MS, ESI: m/z** |
|---|---|---|---|---|
| 26 | 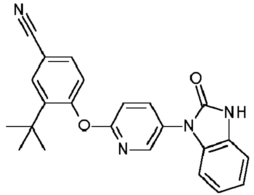 | 3-tert-butyl-4-[[5-(2-oxo-3H-imidazo[4,5-c]pyridin-1-yl)-2-pyridyl] oxy] benzonitrile | 3-(1,1-dimethylethyl )-4-hydroxybenz onitrile (CAS: 4910-04-7) | 385.9 [M+H]⁺ |

### Example 19

### 2-isopropyl-4-[[5-(2-oxo-3H-benzimidazol-1-yl)-2-pyridyl]oxy]benzonitrile

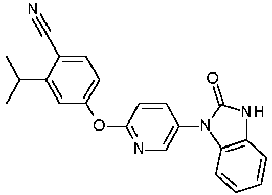

To a solution of 4-hydroxy-2-isopropyl-benzonitrile (CAS: 14114-32-0) (0.200 g, 1.36 mmol) and 3-(6-chloro-3-pyridyl)-1H-benzimidazol-2-one (303 mg, 1.24 mmol) in NMP (6.0 mL), K₂CO₃ (205 mg, 1.48 mmol) was added and the mixture was heated to 200 °C and stirred for 5 h. The mixture was cooled to 25 °C and diluted with EA (50 mL) and washed with H₂O (50 mL), followed by aqueous NaCl solution (50 mL), and dried with anhydrous MgSO₄ to give a residue which was purified by Prep-HPLC to give the title compound (33.0 mg, 88.4 µmol, 7.84% yield) as a white solid. MS (ESI): m/z = 371.2 [M+H]⁺

### Step a) 6-chloro-N-(2-nitrophenyl)pyridin-3-amine

A mixture of 2-chloro-5-iodopyridine (20.0 g, 83.5 mmol), 2-nitroaniline (11.5 g, 83.5 mmol), Pd(OAc)₂ (1.88 g, 8.35 mmol), TEA (8.45 g, 83.5 mmol, 11.6 mL), Cs₂CO₃ (136 g, 418 mmol) and BINAP (1.56 g, 2.51 mmol) in toluene (400 mL) was degassed and purged with N₂ for 3 times, and then the mixture was stirred at 110 °C for 48 h under N₂ atmosphere. The crude reaction mixture was washed with ethyl acetate (100 mL x 3) and brine (100 mL x 3). The aqueous phase was extracted with ethyl acetate (100 mL) again. The combined organic phase was dried with anhydrous Na₂SO₄, filtered and concentrated in vacuum. The crude product was triturated with the mixed solvent (Petroleum ether: Ethyl acetate = 3: 1, 250 mL) to yield the title compound (13 g) was obtained as a red solid. MS (ESI): m/z = 250.0 [M+H]⁺

### Step b) N2-(6-chloro-3-pyridyl)benzene-1,2-diamine

A mixture of 6-chloro-N-(2-nitrophenyl)pyridin-3-amine (12.0 g, 48.1 mmol) and SnCl₂•2H₂O (54.2 g, 240 mmol) in EtOH (200 mL) was stirred at 70°C for 2 h. The reaction mixture was evaporated to dryness. The resulting crude oil was dissolved in EtOAc (250 mL) and treated with NaOH 1M (200 mL). The biphasic system was vigorously stirred for 20 min to allow complete precipitation of the tin salts. The medium was then filtered over diatomaceous earth through a sintered funnel and copiously washed with EtOAc (100mL x 3). The organic layer was washed with brine (300 ml) and the organic finally dried over Na₂SO₄, filtered and the filtrate was concentrated under reduce pressure. The residue was purified with silica gel chromatography column by eluting with PE: EA = 10:1 to 3:1 to give the title compound (8.00 g, 34.2 mmol, 71.1% yield, 93.8 % purity) as brown solid. MS (ESI): m/z = 220.2 [M+H]⁺

### Step c) 3-(6-chloro-3-pyridyl)-1H-benzimidazol-2-one

To a solution of N2-(6-chloro-3-pyridyl)benzene-1,2-diamine (6.40 g, 29.1 mmol) in DCM (300.0 mL) was added pyridine (9.22 g, 117 mmol, 9.41 mL) and stirred at 0 °C. A solution of triphosgene (3.29 g, 11.1 mmol) in DCM (100 mL) was added cautiously over a period of 30 minutes, the mixture was stirred at 0 °C for 1 h and heated to 25 °C for 2 h. The mixture was washed with HCl (0.5 M (aqueous), 150 mL x 2), the organic phase was concentrated under vacuum to give crude product. The aqueous phase was adjusted pH ∼ 8 with NaHCO₃, extracted with ethyl acetate (300 mLx 3) and concentrated under vacuum to give crude product. The combined crude products (6.71 g, 27.3 mmol, 93.8% yield) as white solid was used directly without purification. MS (ESI): m/z = 246.1 [M+H]⁺

In analogy to Example 19, Examples 20-22 were generated using the respective phenol building blocks in the final step.

| **Ex.** | **Structure** | **Systematic Name** | **Building Blocks** | **MS, ESI: m/z** |
|---|---|---|---|---|
| 20 | 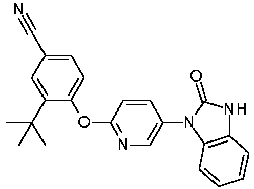 | 3-tert-butyl-4-[[5-(2-oxo-3H-benzimidazol-1-yl)-2-pyridyl] oxy] benzonitrile | 3-(1,1-dimethyl ethyl )-4-hydroxybenz onitrile (CAS: 4910-04-7) | 385.1 [M+H]⁺ |
| 21 | 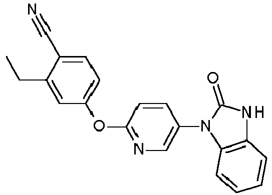 | 2-ethyl-4-[[5-(2-oxo-3H-benzimidazol-1-yl)-2-pyridyl]oxy]benzonitrile | 2-ethyl-4-hydroxybenz onitrile (CAS: 14143-33-0) | 357.1 [M+H]⁺ |
| 22 | 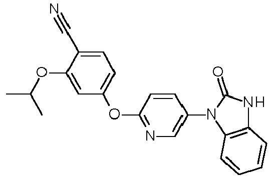 | 2-isopropoxy-4-[[5-(2-oxo-3H-benzimidazol-1-yl)-2-pyridyl] oxy] benzonitrile | **B.2** (4-hydroxy-2-isopropoxy-benzonitrile) | 387.0 [M+H]⁺ |

### Example 30

### 1-[6-(3-methoxy-4-methyl-phenoxy)-3-pyridyl]-3H-imidazo[4,5-b]pyridin-2-one

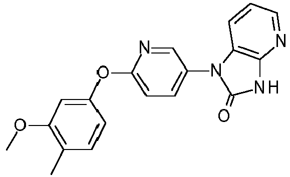

To a solution of 3-N-[6-(3-methoxy-4-methylphenoxy)pyridin-3-yl]pyridine-2,3-diamine (70 mg, 0.18 mmol) in DCM (4 mL) was added triethylamine (0.07 mL, 0.53 mmol) at RT and cooled to 0 °C, followed by the dropwise addition of a solution of triphosgene (26 mg, 0.09 mmol) in DCM (1 mL) at the same temperature and the reaction mixture was stirred at room temperature for 1.5 h. The reaction mixture was diluted with water (20 ml) and extracted with DCM (20 mL x 2). The organic layer was washed with brine (20 mL), dried, filtered and concentrated under reduced pressure. The crude material was then purified by flash chromatography (eluting with a gradient of 0 to 10% MeOH/DCM) to get the title compound (12.0 mg, 19%) as a brown solid. MS (ESI): m/z = 348.9 [M+H]⁺

### Step a) tert-butyl N-[6-(3-methoxy-4-methylphenoxy)pyridin-3-yl]carbamate

To a mixture of 6-(3-methoxy-4-methylphenoxy)pyridin-3-amine (A.3) (100 mg, 0.43 mmol) and di-tert-butyl dicarbonate (0.15 mL, 0.65 mmol) in DCM (3 mL) was added triethylamine (0.2 mL, 1.30 mmol) at 0°C. The mixture was then stirred at 25 °C for 24 h. The reaction mixture was then concentrated in vacuo, giving a residue which was purified via silica gel chromatography (0% to 40% ethyl acetate/hexane) to give tert-butyl N-[6-(3-methoxy-4-methylphenoxy)pyridin-3-yl]carbamate (110 mg, 77%) as a colorless sticky solid. MS (ESI): m/z = 330.8 [M+H]⁺

### Step b) tert-butyl N-[6-(3-methoxy-4-methylphenoxy)pyridin-3-yl]-N-(2-nitropyridin-3-yl)carbamate

To the stirred solution of tert-butyl N-[6-(3-methoxy-4-methylphenoxy)pyridin-3-yl]carbamate (140 mg, 0.42 mmol) in DMF (2 mL), 3-fluoro-2-nitropyridine (61 mg, 0.42 mmol) and cesium carbonate (346 mg, 1.06 mmol) were added. The reaction mixture was then allowed to stir at 80 °C for 2 h. The reaction mixture was diluted with ice cold water (20 mL) and extracted with ethyl acetate (20 mL x 3). The combined organic layer was washed with brine (30 mL), dried, filtered and concentrated under reduced pressure. The crude material thus obtained was purified by flash chromatography (0-50% EtOAc/Hexane) to give tert-butyl N-[6-(3-methoxy-4-methylphenoxy)pyridin-3-yl]-N-(2-nitropyridin-3-yl)carbamate (150 mg, 78%) as yellowish sticky solid. MS (ESI): m/z = 452.7 [M+H]⁺

### Step c) tert-butyl N-(2-aminopyridin-3-yl)-N-[6-(3-methoxy-4-methylphenoxy)pyridin-3-yl]carbamate

To a stirred solution of tert-butyl N-[6-(3-methoxy-4-methylphenoxy)pyridin-3-yl]-N-(2-nitropyridin-3-yl)carbamate (150 mg, 0.33 mmol) in methanol (6 mL) and 1 drop of water, was added zinc dust (152 mg, 2.32 mmol) and NH₄Cl (196 mg, 3.65 mmol) at 25°C and the reaction mixture was stirred at 90 °C for 1 h. The reaction mixture was filtered through celite and the filtrate was evaporated. The residue was diluted with DCM (50 mL) and washed with water (30 mL). The organic layer was dried, filtered and concentrated in vacuo to get tert-butyl N-(2-aminopyridin-3-yl)-N-[6-(3-methoxy-4-methylphenoxy)pyridin-3-yl]carbamate (130 mg, 93%) as a light brown solid. MS (ESI): m/z = 422.7 [M+H]⁺

### Step d) 3-N-[6-(3-methoxy-4-methylphenoxy)pyridin-3-yl]pyridine-2, 3-diamine

To tert-butyl N-(2-aminopyridin-3-yl)-N-[6-(3-methoxy-4-methylphenoxy)pyridin-3-yl]carbamate (130 mg, 0.31 mmol) was added 4 N HCl in dioxane (3 mL) cooled in advance at 0°C and the reaction mixture was stirred at RT for 1 h. The reaction mixture was concentrated under reduced pressure and the residue was triturated and dried under reduced pressure to get HCl salt of 3-N-[6-(3-methoxy-4-methylphenoxy)pyridin-3-yl]pyridine-2,3-diamine (90 mg, 98%) as a light yellowish solid, which was used directly without further purification. (Monitoring by TLC.)

In analogy to Example 30, Examples 31-33 were generated using the respective fluoro-nitropyridine building blocks in Step b).

| **Ex.** | **Structure** | **Systematic Name** | **Building Blocks** | **MS, ESI: m/z** |
|---|---|---|---|---|
| 31 | 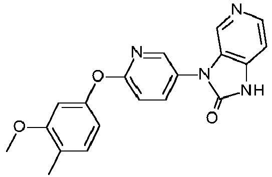 | 3-[6-(3-methoxy-4-methyl-phenoxy)-3-pyndyl]-1H-imidazo[4,5-c] pyridin-2-one | 3-fluoro-4-nitropyridine (CAS: 13505-01-6) | 349.2 [M+H]⁺ |
| 32 | 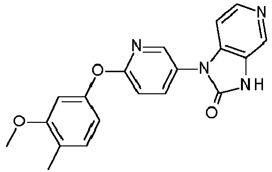 | 1-[6-(3-methoxy-4-methyl-phenoxy)-3-pyridyl]-3H-imidazo[4,5-c] pyridin-2-one | 4-fluoro-3-nitropyridine (CAS: 115812-96-9) | 349.2 [M+H]⁺ |
| 33 | 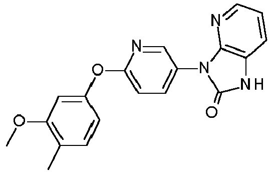 | 3-[6-(3-methoxy-4-methyl-phenoxy)-3-pyridyl]-1H-imidazo[4,5-b]pyridin-2-one | 2-fluoro-3-nitropyridine (CAS: 1480-87-1) | 349.2 [M+H]⁺ |

### Example 34

### 3-[6-(3-methoxy-4-methyl-phenoxy)-3-pytidyl]-1H-benzimidazol-2-one

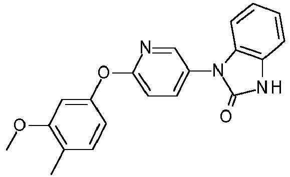

A solution of 1-N-[6-(3-methoxy-4-methylphenoxy)pyridin-3-yl]benzene-1,2-diamine (40 mg, 0.12 mmol) and triethylamine (0.052 mL, 0.37 mmol) in DCM (3 mL) was cooled to 0°C under argon. A solution of triphosgene (18 mg, 0.06 mmol) in DCM (1 mL) was added dropwise and allowed to stir for 30 min keeping the temperature the same. The reaction mass was quenched with water (10 mL) and extracted with DCM (10 mL x 3). The combined organic part was washed with brine (10 ml), dried, filtered and concentrated in vacuo to get crude mass which was purified by flash chromatography, eluting with 10-50% EtOAc in hexane to get the title compound (31 mg, 72%) as an off white solid. MS (ESI): m/z = 348.2 [M+H]⁺

### Step a) 6-(3-methoxy-4-methylphenoxy)-N-(2-nitrophenyl)pyridin-3-amine

A mixture of 6-(3-methoxy-4-methylphenoxy)pyridin-3-amine (**A.3**) (122 mg, 0.53 mmol) and cesium carbonate (432 mg, 1.33 mmol) in 1,4-dioxane (4 mL) was degassed with argon for 5 minutes, followed by the addition of 1-bromo-2-nitrobenzene (107 mg, 0.53 mmol), Pd₂dba₃ (49 mg, 0.053 mmol) and Xantphos (46 mg, 0.079 mmol). The reaction mixture was then sealed and heated at 90°C for 18 h. The reaction mass was filtered through a celite bed and washed with EtOAc (50 mL). The combined organic parts were washed with water (20 mL), brine (20 mL), dried, filtered and concentrated in vacuo to get crude mass which was purified by flash chromatography, eluting with 2-5% EtOAc in hexane to get the title compound (156 mg, 84%) as a brown gum. MS (ESI): m/z = 351.9 [M+H]⁺

### Step b) 1-N-[6-(3-methoxy-4-methylphenoxy)pyridin-3-yl]benzene-1, 2-diamine

A mixture of 6-(3-methoxy-4-methylphenoxy)-N-(2-nitrophenyl)pyridin-3-amine (150 mg, 0.43 mmol), zinc powder (195 mg, 2.99 mmol) and ammonium chloride (251 mg, 4.7 mmol) in methanol (8 mL) and water (0.03 mL) was heated at reflux for 1 h. The reaction mixture was filtered through a sintered funnel and the filtrate was concentrate in vacuo. The residue was diluted with EtOAc (50 mL) and washed with water (20 mL x 2), brine (10 mL), dried, filtered and concentrated in vacuo to get the title compound (130 mg, 95%) as a brown solid. MS (ESI): m/z = 321.9 [M+H]⁺

### Example 38

### 3,5-dimethyl-4-[[5-(2-oxo-1H-imidaz0[4,5-b]pyridin-3-yl)-2-pyridyl]oxy]benzonitrile

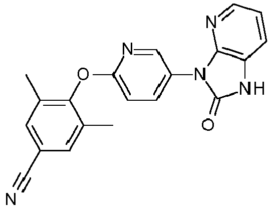

### Library Chemistry Protocol:

To a mixture of 4-[[5-[(3-amino-2-pyridyl)amino]-2-pyridyl]oxy]-3,5-dimethyl-benzonitrile (1 eq.) in THF (0.2 M) was added CDI (3 eq.), pyridine (2.5 eq) in one portion. The mixture was stirred at 65 °C for 3 h. The reaction mixture was concentrated to remove THF. The crude product was purified by prep-HPLC to give the final product (24.5 mg, 1.7 % yield over 5 steps). MS (ESI): m/z = 358.0 [M+H]⁺

### Step a) 3,5-dimethyl-4-[(5-nitro-2-pyridyl)oxy]benzonitrile

To a mixture of 4-hydroxy-3,5-dimethyl-benzonitrile (4.0 mmol, 1.0 eq) and 2-chloro-5-nitropyridine (4.0 mmol, 1.0 eq) in DMF (5.0 mL) was added K₂CO₃ (4 mmol, 1.0 eq) in one portion. The mixture was stirred at 100 °C for 1 h. The mixture was poured into water (10 mL), and then was extracted with ethyl acetate (10 mL × 3). The combined organic phase was concentrated by Speedvac to give crude product which was used for next step directly.

### Step b) 4-[(5-amino-2-pyridyl)oxy]-3,5-dimethyl-benzonitrile

To a solution of 3,5-dimethyl-4-[(5-nitro-2-pyridyl)oxy]benzonitrile (4.0 mmol, 1.00 eq) in MeOH (10 mL) was added Pd(OH)₂ (56 mg, 10.0% purity, 1.00 eq) followed by triethylsilane (4.0 mL). The mixture was stirred at 20 °C for 1 h. The mixture was filtered to remove the Pd(OH)₂, washed with MeOH (10 mL x 3) and condensed by Speedvac to give crude product, which was used for next step directly.

### Step c) 3,5-dimethyl-4-[[5-[(3-nitro-2-pyridyl)amino]-2-pyridyl]oxy]benzonitrile

To a mixture of 4-[(5-amino-2-pyridyl)oxy]-3,5-dimethyl-benzonitrile (1.8 mmol, 1.0 eq) and 2-fluoro-3-nitropyridine (2.7 mmol, 1.5 eq) in NMP (4 mL) was added Cs₂CO₃ (2.7 mmol, 1.5 eq) in one portion. The mixture was stirred at 200 °C for 4 hrs under microwave heating. The mixture was poured into 10 mL of water, and then was extracted with ethyl acetate (10 mL × 3). The combined organic phase was concentrated by Speedvac to give crude product which was used for next step directly.

[In some cases, a modification was used: DMSO, Cs₂CO₃ (1.5 eq), stirred at 100 °C for 16 h, followed by filtration and purification by HPLC].

### Step d) 4-[[5-[(3-amino-2-pyridyl)amino]-2-pyridyl]oxy]-3,5-dimethyl-benzonitrile

To a solution of 3,5-dimethyl-4-[[5-[(3-nitro-2-pyridyl)amino]-2-pyridyl]oxy]benzonitrile (1.00 eq) in MeOH (0.1 M) was added Pd(OH)₂ (10.0% purity, 0.1 eq) followed by triethylsilane (10.0 eq). The mixture was stirred at 20 °C for 1 h.The mixture was filtered and concentrated by Speedvac to give crude product which was used for next step directly.

In analogy to Example 38, Examples 39-41 were generated using the respective phenol building blocks in Step a).

| **Ex.** | **Structure** | **Systematic Name** | **Building Blocks** | **MS, ESI: m/z** |
|---|---|---|---|---|
| 39 | 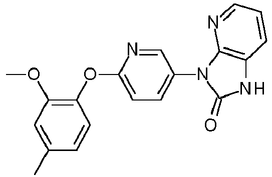 | 3-[6-(2-methoxy-4-methyl-phenoxy)-3-pyridyl]-1H-imidazo[4,5-b]pyridin-2-one | 2-methoxy-4-methylphenol (CAS: 93-51-6) | 349.1 [M+H]⁺ |
| 40 | 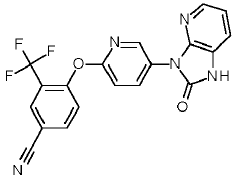 | 4-[[5-(2-oxo-1H-imidazo[4,5-b]pyridin-3-yl)-2-pyridyl]oxy]-3-(trifluoromethyl) benzonitri le | 4-hydroxy-3-(trifluoromet hyl)benzonitr ile (CAS: 124811-71-8) | 398.0 [M+H]⁺ |
| 41 | 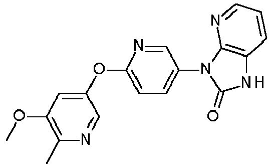 | 3-[6-[(5-methoxy-6-methyl-3-pyridyl)oxy]-3-pyridyl]-1H-imidazo [4,5-b]pyridin-2-one | 5-methoxy-6-methylpyridi n-3-ol (CAS: 1256793-06-2) | 350.1 [M+H]⁺ |

### Example 42

### 1-[6-(3-chloro-4-methyl-phenoxy)-3-pyridyl]-3H-imidazo[4,5-c]pyridin-2-one

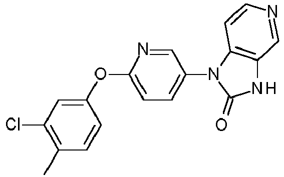

To a solution of N4-[6-(3-chloro-4-methyl-phenoxy)-3-pyridyl]pyridine-3,4-diamine (901 mg, 2.76 mmol) in THF (15 mL) was added CDI (1.34 g, 8.27 mmol) and pyridine (545 mg, 6.89 mmol, 0.556 mL) .The mixture was stirred at 65 °C for 3 h. Remove solvent under reduce pressure. The residue was purified by prep-HPLC (basic condition). The title compound (468 mg, 1.31 mmol, 47.7% yield, 99.1% purity) was obtained as a white solid. MS (ESI): m/z = 353.0 [M+H]⁺

### Step a) 2-(3-chloro-4-methyl-phenoxy)-5-nitro-pyridine

To a mixture of 3-chloro-4-methyl-phenol (428 g, 3.0 mol) and 2-chloro-5-nitro-pyridine (476 g, 3.00 mol) in DMF (5 mL) was added K₂CO₃ (415 mg, 3.00 mmol) in one portion. The mixture was stirred at 100 °C for 1 h. The mixture was cooled to 20 °C and concentrated under reduced pressure at 40 °C. The residue was poured into water (8 mL).The aqueous phase was extracted with ethyl acetate (10 mL x 3). The combined organic phase was dried with anhydrous Na₂SO₄, filtered and concentrated in vacuum. The title compound (820 mg, crude) was obtained as a yellow solid, which was used for the next step without further purification. MS (ESI): m/z = 265.1 [M+H]⁺

### b) 6-(3-chloro-4-methyl-phenoxy)pyridin-3-amine

To a solution of 2-(3-chloro-4-methyl-phenoxy)-5-nitro-pyridine (820 mg, 3.10 mmol) in MeOH (15 mL) was added Pd(OH)₂ (43.5 mg, 310 µmol) and then triethylsilane (3.01 g, 25.9 mmol, 4.13 mL). The mixture was stirred at 20 °C for 2 h. The solution turned to colorless. The mixture was filtered and concentrated by Speedvac. The title compound (701 mg, crude) was obtained as a yellow solid, which was used for the next step without further purification. MS (ESI): m/z = 234.9 [M+H]⁺

### Step c) 6-(3-chloro-4-methyl-phenoxy)-N-(3-nitro-4-pyridyl)pyridin-3-amine

To a mixture of 6-(3-chloro-4-methyl-phenoxy)pyridin-3-amine (701 mg, 2.99 mmol) and 4-chloro-3-nitropyridine (947 mg, 5.97 mmol) in dioxane (15 mL) was added citric acid hydrate (1.26 g, 5.97 mmol, 1.39 mL) in one portion. The mixture was stirred at 80 °C for 16 h. The mixture was cooled to 20 °C and concentrated in reduced pressure. The residue was diluted with saturated Na₂CO₃ aqueous (30 mL).The aqueous phase was extracted with ethyl acetate (30 mL x 3).The combined organic phase was dried with anhydrous Na₂SO₄, filtered and concentrated under vacuum. The title compound (911 mg, crude) was obtained as a white solid, which was used for the next step without further purification. MS (ESI): m/z = 357.2 [M+H]⁺

### Step d) N4-[6-(3-chloro-4-methyl-phenoxy)-3-pyridyl]pyridine-3,4-diamine

To a solution of 6-(3-chloro-4-methyl-phenoxy)-N-(3-nitro-4-pyridyl)pyridin-3-amine (911 mg, 2.55 mmol) in MeOH (20 mL) was added triethylsilane (2.52 g, 21.7 mmol, 3.47 mL) and then Pd(OH)₂ (35.9 mg, 255 µmol). The mixture was stirred at 20 °C for 1 h. The reaction mixture was filtered and concentrated under reduced pressure. The title compound (892 mg, crude) was obtained as a light yellow solid, which was used for the next step without further purification. MS (ESI): m/z = 327.1 [M+H]⁺

### Example 43

### 9-[6-(3-methoxy-4-methyl-phenoxy)-3-pyridyl]-7H-purin-8-one

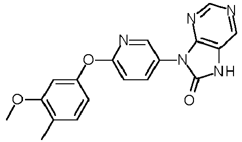

To a solution of N4-[6-(3-methoxy-4-methyl-phenoxy)-3-pyridyl]pyrimidine-4,5-diamine (240 mg, 0.742 mmol) in MeCN (5.00 mL) was added CDI (241 mg, 1.48 mmol), the formed mixture was stirred at 40 °C for 4 h. The mixture was concentrated under vacuum to give crude product which was purified by prep-HPLC and lyophilized to give impure title compound (56.0 mg, 0.160 mmol, 21.6% yield) which was re-purified by prep-HPLC and lyophilized to give the title compound (19.5 mg, 55.3 µmol, 34.51% yield, 98.9% purity) as white solid. MS (ESI): m/z = 350.1 [M+H]⁺

### Step a) 5-bromo-N-[6-(3-methoxy-4-methyl-phenoxy)-3-pyridyl]pyrimidin-4-amine

To a mixture of 6-(3-methoxy-4-methylphenoxy)pyridin-3-amine (**A.3**) (1.00 g, 4.34 mmol) and 4-chloro-5-bromopyrimidine (840 mg, 4.34 mmol) in NMP (4.00 mL) was added TsOH·H₂O (826 mg, 4.34 mmol). The mixture was stirred at 80°C for 2 h. The reaction mixture was filtered, water was added (20.0 mL) and the mixture was extracted with ethyl acetate (20.0 mL x 3). The combined organic layers were washed with brine (30.0 mL), dried over Na₂SO₄, filtered and concentrated under reduced pressure to give a residue. The residue was purified by column chromatography (eluting with Petroleum ether/Ethyl acetate = 100 to 0% gradient) and concentrated under vacuum to give the title compound (1.50 g, 3.87 mmol, 89.2% yield) was obtained as a yellow solid. MS (ESI): m/z = 387.0 [M+H]⁺

### Step b) N4-[6-(3-methoxy-4-methyl-phenoxy)-3-pyridyl]-N5-[(4-methoxyphenyl) methyl]pyrimidine-4,5-diamine

To a mixture of 5-bromo-N-[6-(3-methoxy-4-methyl-phenoxy)-3-pyridyl]pyrimidin-4-amine (1.50 g ,3.87 mmol) and (4-methoxyphenyl) methanamine (797 mg, 5.81 mmol, 752 µL) in t-Amyl-OH (15.0 mL) was added tBuXPhos Pd G3 (154 mg, 194 µmol), tBuONa (2.00 M, 5.81 mL) in one portion under N₂. The mixture was stirred at 90 °C for 12 h under N₂. To the reaction mixture was added water (50.0 mL), and extracted with ethyl acetate (50.0 mL x 3). The combined organic layers were washed with brine (100 mL), dried over Na₂SO₄, filtered and concentrated under reduced pressure to give a residue which was purified by column chromatography (eluting with Petroleum ether/Ethyl acetate = 100 to 0% gradient) to give the title compound (250 mg, 564 µmol, 14.6% yield) as a brown liquid. MS (ESI): m/z = 444.2 [M+H]⁺

### Step c) N4-[6-(3-methoxy-4-methyl-phenoxy)-3-pyridyl]pyrimidine-4,5-diamine

To a mixture of N4-[6-(3-methoxy-4-methyl-phenoxy)-3-pyridyl]-N5-[(4-methoxyphenyl)methyl]pyrimidine-4,5-diamine (250 mg, 564 µmol) in DCM (10.0 mL) was added TFA (4.00 mL) in one portion under N₂. The mixture was stirred at 20 °C for 2 h. The mixture was poured into saturated sodium carbonate aqueous solution (30.0 mL) and extracted with DCM (30.0 mL x 3). Then the combined organic phase was dried over with anhydrous Na₂SO₄, filtered and concentrated to give the title compound (240 mg, crude) as brown oil, which was used directly without further purification. MS (ESI): m/z = 324.1 [M+H]⁺

### Example 48

### 3-[6-(3-methoxy-4-methyl-phenoxy)-3-pyridyl]-1H-imidazo[4,5-b]pyrazin-2-one

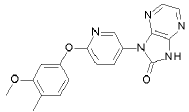

To a mixture of N3-[6-(3-methoxy-4-methyl-phenoxy)-3-pyridyl]pyrazine-2,3-diamine (213 mg, 660 µmol) in THF (6 mL) was added CDI (641 mg, 3.96 mmol), pyridine (313 mg, 3.96 mmol) in one portion. The mixture was stired at 65 °C for 2 h. The reaction mixture was concentrated to remove THF. The residue was purified by prep-HPLC. The title compound was obtained as a yellow solid (82.5 mg, 35.6 % yield, 90% purity), followed by re-purification by prep-HPLC, to obtain the title compound as a yellow solid (32.3 mg, 14.3% yield). MS (ESI): m/z = 350.2 [M+H]⁺

### Step a) 3-chloro-N-[6-(3-methoxy-4-methyl-phenoxy)-3-pyridyl]pyrazin-2-amine

To a solution of 6-(3-methoxy-4-methylphenoxy)pyridin-3-amine (**A.3**) (2.00 mmol) in THF (18 mL) was added a solution of NaHMDS (4.0 mL, 1.0 M in THF, 2.0 eq.) drop-wise at 0°C under protection of N₂. The reaction mixture was warmed to 30 °C and stirred for 8 h. 2,3-dichloropyrazine (4.00 mmol, 2.0 eq) was added into above solution. The reaction mixture was stirred at 30 °C for another 16 h. The residue was purified by flash silica gel chromatography. The title compound was obtained as yellow oil (498 mg, 72.7% yield).

### Step b) N-[6-(3-methoxy-4-methyl-phenoxy)-3-pyridyl]tetrazolo[1,5-a]pyrazin-8-amine

To a mixture of 3-chloro-N-[6-(3-methoxy-4-methyl-phenoxy)-3-pyridyl]pyrazin-2-amine (200 mg, 583 µmol) in DMF (2 mL) was added NaN₃ (130 mg, 2.00 mmol) in one portion at 25 °C under N₂. The mixture was heated to 100 °C and stirred for 20 h. The mixture was cooled to 25 °C and poured into ice-water (w/w = 1/1) (6 mL) and stirred for 10 min. The aqueous phase was extracted with ethyl acetate (20 mL x 2). The combined organic phase was washed with brine (20 mL x 2), dried with anhydrous Na₂SO₄, filtered and concentrated in vacuum. The title compound (200 mg, crude) was obtained as a yellow solid, which was used directly without further purification. MS (ESI): m/z = 350.2 [M+H]⁺

### Step c) N3-[6-(3-methoxy-4-methyl-phenoxy)-3-pyridyl]pyrazine-2,3-diamine

To a mixture of N-[6-(3-methoxy-4-methyl-phenoxy)-3-pyridyl]tetrazolo[1,5-a]pyrazin-8-amine (190 mg, 489 µmol) in H₂O (10 mL), 1-butanol (10 mL) and EtOH (10 mL) was added Fe (1.37 g, 24.5 mmol) and NH₄Cl (2.62 g, 49.0 mmol) in one portion at 25 °C under N₂. The mixture was heated to 100 °C and stirred for 13 h. A further addition of Fe (4.10 g, 73.4 mmol) and NH₄Cl (7.85 g, 147 mmol) was made in one portion. The mixture was stirred at 120 °C for 5 h. The mixture was cooled to 25 °C. The mixture was filtered and the filtrate was concentrated in reduced pressure at 40 °C. The residue was poured into ice-water (w/w = 1/1) (20 mL) and stirred for 10 min. The aqueous phase was extracted with ethyl acetate (30 mL x 2).The combined organic phase was washed with brine (30 mL x 2), dried with anhydrous Na₂SO₄, filtered and concentrated in vacuum. The title compound (170 mg, crude) was obtained as yellow oil, which was used directly without further purification. MS (ESI): m/z = 324.2 [M+H]⁺

### Example 52

### 3-isopropyl-4-[3-(2-oxo-1H-imidazo[4,5-b]pyridin-3-yl)cyclobutoxy]benzonitrile

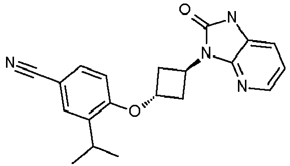

To a solution of rac-3-((1r,3r)-3-hydroxycyclobutyl)-1,3-dihydro-2H-imidazo[4,5-b]pyridin-2-one (50 mg, 244 µmol) in dry DMF (1.5 mL) was added NaH (20.5 mg, 512 µmol) and the reaction mixture was stirred at room temperature for 15 min followed by addition of 4-chloro-3-isopropylbenzonitrile (**C.2**) (52.5 mg, 292 µmol). The reaction mixture was stirred at 80 °C for 60 min, then at 110 °C for 30 min under microwave radiation. The reaction mixture was then stirred at 110 °C for another 16 h. The reaction mixture was quenched by addition of few drops of sat. aq. NH₄Cl solution and the crude solution was directly submitted for reversed-phase HPLC purification to yield 21.4 mg of the title compound. MS (ESI): m/z = 349.2 [M+H]⁺

### Step b) rac-1-(2-bromopyridin-3-yl)-3-((1r,3r)-3-hydroxycyclobutyl)urea

To a solution of 2-bromopyridin-3-amine (0.5 g, 2.89 mmol) in dry THF (10 mL) under an inert atmosphere was added DIPEA (392 mg, 530 µl, 3.03 mmol) and phenyl carbonochloridate (475 mg, 382 µl, 3.03 mmol), the reaction mixture was stirred at room temperature for 4 h. Addition of rac-(1r,3r)-3-aminocyclobutan-1-ol hydrochloride (CAS: 1205037-95-1) (375 mg, 3.03 mmol) and DIPEA (784 mg, 1.06 ml, 6.07 mmol), the reaction mixture was then stirred at 70 °C for 16 h. Reaction mixture was diluted with ethyl acetate and extracted with aq. Na₂CO₃ 2 M solution, the organic phase was collected and the aqueous phase was back-extracted with ethyl acetate. The combined organic phase were dried over sodium sulfate and evaporated down to dryness. The crude material was purified by flash chromatography, eluting with a mixture of dichloromethane and methanol (0% to 10%) to yield 695 mg of the title compound. MS (ESI): m/z = 288.1 [M+H]⁺

### Step c) rac-3-((1r,3r)-3-hydroxycyclobutyl)-1,3-dihydro-2H-imidazo[4,5-b]pyridin-2-one

To a suspension of rac-1-(2-bromopyridin-3-yl)-3-((1r,3r)-3-hydroxycyclobutyl)urea (650 mg, 2.27 mmol) in dry dioxane (15 mL) under an inert atmosphere was added DPPF (126 mg, 227 µmol), Pd₂(dba)₃ (104 mg, 114 µmol) and sodium tert-butoxide (546 mg, 5.68 mmol). The reaction mixture was stirred at r.t for 10 min followed by stirring at 90 °C for 1 h. The reaction mixture was poured into a mixture of ethyl acetate and aq. NaHCO₃ 1 M and the mixture was stirred for 5 min. The bi-phasic mixture was then transferred into a separating funnel for extraction. The organic phase was collected and the aqueous phase was back-extracted with ethyl acetate. The combined organic phases were dried over sodium sulfate and evaporated down to dryness. The crude material was purified by flash chromatography, eluting with a mixture of dichloromethane and methanol (0% to 10%) to yield 305mg of the title compound. MS (ESI): m/z = 206.1 [M+H]⁺

### Example 53

### 1-[3-(3-methoxy-4-methyl-phenoxy)cyclobutyl]-3H-imidazo[4,5-c]pyridin-2-one

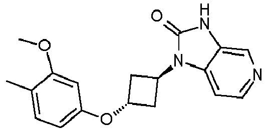

To a suspension of rac-N4-((1r,3r)-3-(3-methoxy-4-methylphenoxy)cyclobutyl)pyridine-3,4-diamine (100 mg, 334 µmol) in a mixture of CH₃CN (1 mL)/DMF (0.2 mL) was added di(1H-imidazol-1-yl)methanone (56.9 mg, 351 µmol) and the reaction mixture was then stirred at room temperature for 1 h. The reaction was then stirred at 50 °C for 1.5 h. A further addition of CDI (114 mg, 701 µmol) was made, and the reaction mixture was stirred at 50 °C for 16 h. Volatiles were removed in vacuo and the crude residue was directly purified by reversed-phase HPLC to yield 48.3 mg of the title compound. MS (ESI): m/z = 206.1 [M+H]⁺

### Step a) tert-butyl((1r,3r)-3-(3-methoxy-4-methylphenoxy)cyclobutyl)carbamate

To a solution of tert-butyl ((1s,3s)-3-hydroxycyclobutyl)carbamate (CAS: tert-butyl ((1s,3s)-3-hydroxycyclobutyl)carbamate) (0.75 g, 4.01 mmol) in dry CH₂Cl₂ (20 mL) was added 3-methoxy-4-methylphenol (609 mg, 4.41 mmol), triphenylphosphine (1.16 g, 4.41 mmol) and DIAD (891 mg, 857 µL, 4.41 mmol). The reaction mixture was then stirred for 18 h at room temperature. A further addition of triphenylphosphine (525 mg, 2.0 mmol) and DIAD (405 mg, 389 µL, 2.0 mmol) was made, and the reaction mixture was then stirred at room temperature for another 18 h. Reaction mixture was diluted with dichloromethane and extracted with aq. NaHCO₃ 1 N solution, the organic phase was collected and the aqueous phase was back-extracted with dichloromethane. The combined organic phases were dried over sodium sulfate and evaporated down to dryness. The crude material was purified by flash chromatography, eluting with a mixture of heptane and ethyl acetate (5% to 20%) to yield 690 mg of a product contaminated with the phenol. This was purified by SFC to yield the title compound (389 mg). MS (ESI): m/z = 308.4 [M+H]⁺

### Step b) (1r,3r)-3-(3-methoxy-4-methylphenoxy)cyclobutan-1-amine; hydrochloride salt

To a solution of tert-butyl ((1r,3r)-3-(3-methoxy-4-methylphenoxy)cyclobutyl)carbamate (1.03 g, 3.35 mmol) in dioxane (5 mL) was added HCl 4.0 M solution in dioxane (5.03 mL, 20.1 mmol) and the reaction mixture was stirred at room temperature for 18 h. Volatiles were removed in vacuo to yield the title compound (805 mg, crude) which was used without further purification. MS (ESI): m/z = 208.2 [M+H]⁺

### Step c) N-((1r,3r)-3-(3-methoxy-4-methylphenoxy)cyclobutyl)-3-nitropyridin-4-amine

To a solution of (1r,3r)-3-(3-methoxy-4-methylphenoxy)cyclobutan-1-amine hydrochloride (530 mg, 2.17 mmol) in dry DMF (10 mL) was added DIPEA (703 mg, 949 µL, 5.44 mmol) and 4-chloro-3-nitropyridine (379 mg, 2.39 mmol). The reaction mixture was then stirred at 80 °C for 18 h. Volatiles were removed in vacuo, the crude residue was partitioned between ethyl acetate and aq. NaHCO₃ 1 M. The organic phase was collected and the aqueous phase was back-extracted with ethyl acetate. The combined organic phases were dried over sodium sulfate and evaporated down to dryness to yield 752 mg of the title compound which was used directly, without further purification.

### Step d) rac-N4-((1r,3r)-3-(3-methoxy-4-methylphenoxy)cyclobutyl)pyridine-3,4-diamine

To a solution of N-((1r,3r)-3-(3-methoxy-4-methylphenoxy)cyclobutyl)-3-nitropyridin-4-amine (716 mg, 2.17 mmol) in acetic acid (7 mL) was added iron (425 mg, 7.61 mmol) and the reaction mixture was stirred at 50 °C for 1.5 h. (After 30 min at 50 °C, a thick precipitate formed which disrupted stirring. Dilution with acetic acid (2.5 mL) was needed to resume stirring.) Volatiles were removed in vacuo, the crude residue was suspended in dichloromethane and an aqueous solution of ammonia 6 N was added. The bi-phasic mixture was vigorously stirred for 15 min, control of the aqueous phase pH indicated 6-7 so the pH was basified by addition of concentrated aqueous NaOH solution until pH roughly 10-11. A precipitate formed which was removed by filtration over a pad of celite, the filter pad was washed with dichloromethane and the bi-phasic filtrate was then transfered into a separating funnel. After extraction an emulsion made the phase separation difficult so it was removed by filtration over a pad of celite and the organic phase was collected. The aqueous phase was back-extracted with dichloromethane twice and the combined organic phases were dried over sodium sulfate and evaporated down to dryness. The residue was purified by flash chromatography, eluting with a mixture of dichloromethane and methanol (1% to 20%, then 20% of a solution of 3% Et₃N in methanol), to yield the title compound (698 mg). MS (ESI): m/z = 300.3 [M+H]⁺

### Example 47

### 1-[6-[4-methyl-3-(trifluoromethoxy)phenoxy]-3-pyridyl]-3H-imidazo[4,5-c]pyridin-2-one

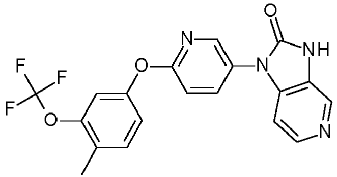

To a solution of 1-[6-[4-bromo-3-(trifluoromethoxy)phenoxy]-3-pyridyl]-3H-imidazo[4,5-c]pyridin-2-one (35.0 mg, 0.070 mmol) in 1,4-dioxane (2 mL) was added K₂CO₃ (31.1 mg, 0.220 mmol), trimethylboroxine (94.0 mg, 0.370 mmol) and [1,1'-bis(diphenylphosphino)ferrocene]dichloropalladium(II) (7.0 mg, 0.010 mmol). The mixture was stirred for 6 hr at 110 °C under N₂. The reaction was filtered and the filtrate was concentrated to dryness. The residue was purified by Prep-HPLC (FA) and lyophilized (twice) to give 1-[6-[4-methyl-3-(trifluoromethoxy)phenoxy]-3-pyridyl]-3H-imidazo[4,5-c]pyridin-2-one (4.3 mg, 0.010 mmol, 13.8% yield) as light grey solid. MS (ESI): m/z = 403.1 [M+H]⁺

### Step a) 1-[6-[4-bromo-3-(trifluoromethoxy)phenoxy]-3-pyridyl]-3H-imidazo[4,5-c]pyridin-2-one

To a solution of 4-bromo-3-(trifluoromethoxy)phenol (100 mg, 0.390 mmol) in NMP (1.5 mL) was added 1-(6-chloro-3-pyridyl)-3H-imidazo[4,5-c]pyridin-2-one (**Example 16, Step c**) (115 mg, 0.470 mmol) and potassium carbonate (80.7 mg, 0.580 mmol). The mixture was stirred at 200 °C for 2 h in a sealed tube under microwave. The mixture was combined with three other batches, and was filtered and the filtrate was purified by Prep-HPLC (FA) and lyophilized to give 1-[6-[4-bromo-3-(trifluoromethoxy)phenoxy]-3-pyridyl]-3H-imidazo[4,5-c]pyridin-2-one (40 mg, 0.090 mmol, 22% yield) as light brown solid. MS (ESI): m/z = 466.9 [M+H]⁺

### Synthesis of Building Blocks

### Example A.1

### 6-((2H-spiro[benzofuran-3,1'-cyclopropan]-4-yl)oxy)pyridin-3-amine

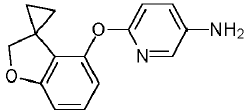

To a solution of 2-((2H-spiro[benzofuran-3,1'-cyclopropan]-4-yl)oxy)-5-nitropyridine (894 mg, 2.99 mmol) in methanol (25 mL) under an inert atmosphere was added Pd/C 10% (w/w 10%) (88 mg, 827 µmol) and ammonium formate (1.88 g, 29.9 mmol, Eq: 10), the reaction mixture was then stirred at 50°C for 3 h. Pd catalyst was removed by filtration over a pad of celite, the filter pad was washed with methanol twice and the filtrate was evaporated down to dryness. The crude residue was partitioned between ethyl acetate and aq. Na₂CO₃ 1 M solution, the organic phase was collected and the aqueous phase was back-extracted with ethyl acetate. The combined organic phases were dried over sodium sulfate and evaporated down to dryness to yield 785 mg of the crude title compound which was used without further purification. MS (ESI): m/z = 255.2 [M+H]⁺

### Step a) 2-((2H-spiro[benzofuran-3,1'-cyclopropan]-4-yl)oxy)-5-nitropyridine

To a solution of 2H-spiro[benzofuran-3,1'-cyclopropan]-4-ol **Example B.1** (500 mg, 3.08 mmol) in DMF (15 mL) was added Cs₂CO₃ (1.0 g, 3.08 mmol) followed by addition of 2-fluoro-5-nitropyridine (438 mg, 3.08 mmol), the reaction mixture was then stirred at 80 °C for 3 h. The reaction mixture was diluted with ethyl acetate and extracted with aq. NaHCO₃ 1 M solution, the organic phase was collected and the aqueous phase was back-extracted with ethyl acetate. The combined organic phases were dried over sodium sulfate and evaporated down to dryness to yield 894 mg of the crude desired product which was used without further purification. MS (ESI): m/z = 285.2 [M+H]⁺

### Example A.2

### 6-((2H-spiro[benzofuran-3,1'-cyclopropan]-4-yl)oxy)pyridin-3-amine

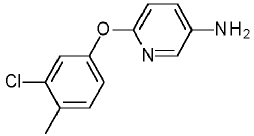

To a solution of 2-(3-chloro-4-methylphenoxy)-5-nitropyridine (4.94 g, 17.7 mmol) in methanol (80 mL) under an inert atmosphere was added Pt/C 5% (500 mg, 2.56 mmol) and ammonium formate (11.2 g, 177 mmol), the reaction was then stirred at 70 °C for 2 h. Pt catalyst was removed by filtration over a pad of celite, the filter pad was washed with methanol and the filtrate concentrated in vacuo. The crude residue was partitioned between ethyl acetate and aq. NaHCO₃ 1 M solution, the organic phase was collected and the aqueous phase was back-extracted with ethyl acetate. The combined organic phases were dried over sodium sulfate and evaporated down to dryness to yield 4.64 g of the title compound as a crude product. This could be carried directly into the next step, or could be further purified by flash chromatography eluting with a mixture of heptane and ethyl acetate (5% to 70%). MS (ESI): m/z = 235.2 [M+H]⁺

### Step a) 2-(3-chloro-4-methylphenoxy)-5-nitropyridine

To a solution of 3-chloro-4-methylphenol (2.5 g, 17.5 mmol) in dry DMF (40 mL) was added NaH (701 mg, 17.5 mmol) by portions and the reaction mixture was then stirred at RT for 10 min followed by addition of 2-chloro-5-nitropyridine (2.78 g, 17.5 mmol). The reaction mixture was then stirred at RT for 1 h. Reaction was quenched by addition of few drops of sat. aq. NH₄Cl solution, diluted with ethyl acetate and extracted with aq. NaHCO₃ 1M solution. The organic phase was collected and the aqueous phase was back-extracted with ethyl acetate. The combined organic phases were dried over sodium sulfate and evaported down to dryness to give 4.94 g of the title compound which was used without further purification. MS (ESI): m/z = 265.2 [M+H]⁺

### Example A.3

### 6-(3-methoxy-4-methyl-phenoxy)pyridin-3-amine

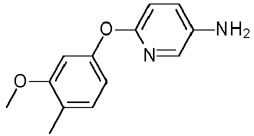

To a solution of 2-(3-methoxy-4-methylphenoxy)-5-nitropyridine (9.87 g, 34.1 mmol) in methanol (200 mL) under an inert atmosphere was added Pd/C 10% (10% w/w) (987 mg, 9.27 mmol) by portions followed by addition of ammonium formate (17.2 g, 273 mmol) by portions. The reaction mixture was then stirred at RT for 2 h. The palladium catalyst was removed by filtration over a pad of celite, the filter pad was washed twice with methanol and the filtrate was concentrated in vacuo. The crude residue was dissolved in ethyl acetate and extracted with aq. NaHCO₃ 1 M, the organic phase was collected and the aqueous phase was back-extracted with ethyl acetate. The combined organic phases were dried over sodium sulfate and eavaporated down to dryness. The crude material was purified by flash chromatography eluting with a mixture of heptane and ethyl acetate (5% to 60%), to yield the title compound (7.25g). MS (ESI): m/z = 231.2 [M+H]⁺

### Step a) 2-(3-methoxy-4-methylphenoxy)-5-nitropyridine

To a solution of 3-methoxy-4-methylphenol (4.97 g, 36 mmol) in dry DMF (100 mL) was added 2-chloro-5-nitropyridine (5.7 g, 36 mmol) and Cs₂CO₃ (12.9 g, 39.5 mmol). The reaction mixture was stirred at 80 °C for 2 h. Volatiles were removed in vacuo and the crude residue was partitioned between ethyl acetate and water, the organic phase was collected and the aqueous phase was back-extracted with ethyl acetate. The combined organic phases were washed with brine, dried over sodium sulfate and evaported down to dryness to yield the title compound (9.87 g, crude) which was used without further purification. MS (ESI): m/z = 261.2 [M+H]⁺

### Example A.4

### 3-fluoro-4-spiro[2H-benzofuran-3,1'-cyclopropane]-4-yloxy-aniline

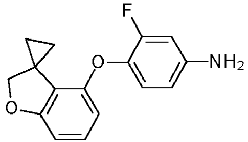

To a solution of compound 4-(2-fluoro-4-nitro-phenoxy)spiro[2H-benzofuran-3,1'-cyclopropane] (680 mg, 2.26 mmol) in THF (10 mL) was added Pd/C (100 mg, 10 % purity) under N₂, and then the mixture was stirred under H₂ (15 psi) at 25 °C for 10 h. The reaction mixture was filtered and the filtrate was concentrated under vacuum to give the title compound (624 mg, 1.82 mmol, 80.6% yield) as a yellow oil. MS (ESI): m/z = 272.0 [M+H]⁺

### Step a) 4-(2-fluoro-4-nitro-phenoxy)spiro[2H-benzofuran-3,1'-cyclopropane]

To a solution of 3,4-difluoronitrobenzene (350 mg, 2.20 mmol, 243 µL) and **Example B.1** (357 mg, 2.20 mmol) in DMF (5 mL) was added K₂CO₃ (608 mg, 4.40 mmol), and the mixture was stirred at 60 °C for 3 h. The mixture was diluted with water (20 mL) and extracted with ethyl acetate (30 mL x 3). The organic phase was dried over sodium sulfate, filtered and concentrated under vacuum to give the title compound (680 mg, crude) as a yellow solid which was used directly without further purification. MS (ESI): m/z = 302.0 [M+H]⁺

In analogy to Example A.4, Examples A.4, A.5 and A.12 of the following table were generated, using the respective building blocks in Step a)

| **Ex.** | **Structure** | **Systematic Name** | **Building Blocks** | **MS, ESI: m/z** |
|---|---|---|---|---|
| A.5 | 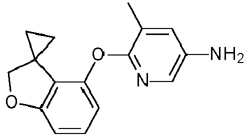 | 5-methyl-6-spiro[2H-benzofuran-3,1'-cyclopropane]-4-yloxy-pyridin-3-amine | **B.1** and 2-chloro-3-methyl-5-nitropyridine CAS: 22280-56-4 | 269.0 [M+H]⁺ |
| A.12 | 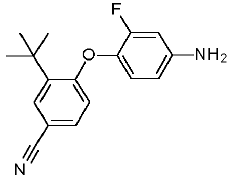 | 4-(4-amino-2-fluoro-phenoxy)-3-tert-butyl-benzonitrile | 3-(1,1-dimethylethyl)-4-hydroxybenzon itrile and 3,4-difluoronitrobe nzene | 285.2 [M+H]⁺ |

### Example A.6

### 4-(4-amino-2-fluoro-phenoxy)-2-isopropyl-benzonitrile

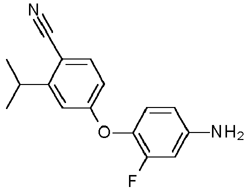

To a solution of 4-(2-fluoro-4-nitro-phenoxy)-2-isopropyl-benzonitrile (710 mg, 2.36 mmol) in ethanol (8 mL) /water (2 mL) was added zinc (773 mg, 11.8 mmol) and ammonium chloride (632 mg, 11.8 mmol). The mixture was stirred at 25°C for 2 h, filtered and the filtrate was concentrated to give the residue. The residue was dissolved in EtOAc/EtOH (3:1). The mixture was filtered and the filtrate was concentrated to give 4-(4-amino-2-fluoro-phenoxy)-2-isopropyl-benzonitrile (550 mg, 2.03 mmol, 86.1% yield) as a yellow oil. MS (ESI): m/z = 271.6 [M+H]⁺

### Step a) 4-(2-fluoro-4-nitro-phenoxy)-2-isopropyl-benzonitrile

To a mixture of 4-chloro-3-fluoronitrobenzene (CAS: 350-31-2) (436 mg, 2.48 mmol) in MeCN (5.3 mL) was added potassium carbonate (1029 mg, 7.44 mmol) and 4-hydroxy-2-isopropyl-benzonitrile (400 mg, 2.48 mmol) at 25 °C. Then the mixture was stirred at 90 °C for 10 h. To the mixture was added EtOAc. The mixture was filtered and the filtrate was concentrated to give the residue, which was purified by flash chromatography (petroleum ether/ethyl acetate = 20:1 to 5:1 gradient,) and concentrated to give 4-(2-fluoro-4-nitro-phenoxy)-2-isopropyl-benzonitrile (690 mg, 2.3 mmol, 92.6% yield) as a colorless oil. MS (ESI): m/z = 301.1 [M+H]⁺

In analogy to Example A.6, Examples A.7 to A.9 of the following table were generated:

| **Ex.** | **Structure** | **Systematic Name** | **Building Blocks** | **MS, ESI: m/z** |
|---|---|---|---|---|
| A.7 | 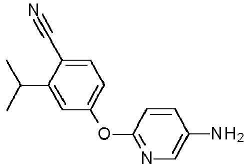 | 4-[(5-amino-2-pyridyl)oxy]-2-isopropyl-benzonitrile | 4-hydroxy-2-isopropyl-benzonitrile (CAS: 14114-32-0) and 2-chloro-5-nitropyridine | 254.1 [M+H]⁺ |
| A.8 | 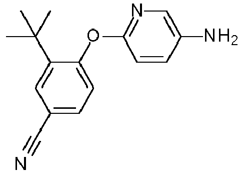 | 4-[(5-amino-2-pyridyl)oxy]-3-tert-butyl-benzonitrile | 3-tert-butyl-4-hydroxybenzonitrile and 2-chloro-5-nitropyridine | 268.1 [M+H]⁺ |
| A.9 | 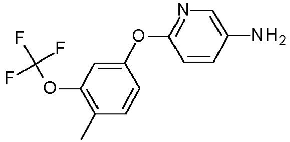 | 6-[4-methyl-3-(trifluoromethoxy)phe noxy]pyridin-3-amine | 4-methyl-3-(trifluorometho xy)phenol (CAS: 1261622-04-1) and 2-chloro-5-nitropyridine | 284.9 [M+H]⁺ |

### Example A.10

### 5-fluoro-6-(3-methoxy-4-methyl-phenoxy)pyridin-3-amine

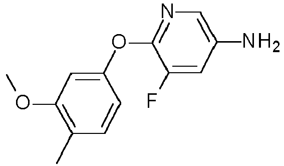

To a solution of 3-fluoro-2-(3-methoxy-4-methyl-phenoxy)-5-nitro-pyridine (4.0 g, 12.2 mmol) and Pd(OH)₂ (3.40 g, 24.2 mmol) in MeOH (100 mL) was added triethylsilane (14.6 g, 125 mmol) under N₂. The mixture was stirred at 25 °C for 17 h. This reaction was concentrated in reduced pressure at 50 °C. The residue was washed with TMBE (50 mL). The residue was discarded. The solution was concentrated in reduced pressure at 40 °C. The residue was washed with PE (100 mL) and filtered to afford the title compound (2.6 g, 9.43 mmol, 77.1 % yield) as a blue solid, which was used for next step directly without purification. MS (ESI): m/z = 249.1 [M+H]⁺

### Step a) 3-fluoro-2-(3-methoxy-4-methyl-phenoxy)-5-nitro-pyridine

To a solution of 3-methoxy-4-methyl-phenol (1.5 g, 10.9 mmol) and 2-chloro-3-fluoro-5-nitropyridine (CAS: 1079179-12-6) (1.92 g, 10.9 mmol) in MeCN (45 mL) was added K₂CO₃ (4.50 g, 32.6 mmol). The reaction was stirred at 85 °C for 16 h. The solution was filtered, and concentrated in vacuum. The residue was purified by flash silica gel chromatography. The title compound (4 g, 12.2 mmol, 56.3 % yield) was obtained as a yellow solid. MS (ESI): m/z = 279.1 [M+H]⁺

In analogy to Example A.10, Examples A.11 of the following table were generated:

| **Ex.** | **Structure** | **Systematic Name** | **Building Blocks** | **MS, ESI: m/z** |
|---|---|---|---|---|
| A.11 | 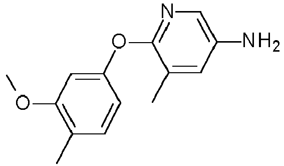 | 5-methyl-6-(3-methoxy-4-methyl-phenoxy)pyridin-3-amine | 2-chloro-3-methyl-5-nitropyridine (CAS: 2-chloro-3-methyl-5-nitropyridine) | 254.1 [M+H]⁺ |

### Example B.1

### spiro[2H-benzofuran-3,1'-cyclopropane]-4-ol

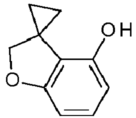

Synthesis as reported in e.g. WO2012/76877 A1, 2012**.** (CAS: 1311139-79-3)

### Example B.2

### 4-hydroxy-2-isopropoxy-benzonitrile

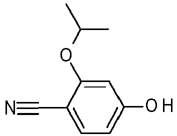

To a solution of 4-bromo-2-isopropoxybenzonitrile (Fluorochem, 1369898-82-7) (0.50 g, 2.08 mmol) in dioxane (5 mL) and H₂O (2 mL) was added KOH (257 mg, 4.58 mmol), t-Bu XPhos (177 mg, 417 µmol) and Pd₂(dba)₃ (191 mg, 208 µmol) in one portion under N₂. The mixture was stirred at 100 °C for 5 h. The reaction mixture was filtered and then was added water (50 mL) and 1 M aq. HCl to adjust pH to 4. The aqueous solution was extracted with DCM (50 mL x 6). The combined organic layers were washed with brine (50 mL), dried over Na₂SO₄, filtered and concentrated under reduced pressure to give a residue which was purified by column chromatography (SiO₂, Petroleum ether/Ethyl acetate = 1:0 to 0:1 gradient) to give the title compound (98.3 mg, 180 µmol, 78 % yield, 93 % purity) as a yellow solid. MS (ESI): m/z = 178.2 [M+H]⁺

### Example C.1

### methyl N-(4-chloropyrimidin-5-yl)carbamate

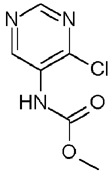

To a solution of 4-chloropyrimidin-5-amine (1500 mg, 11.6 mmol) in pyridine (10 mL) was added methyl chloroformate (1.79 mL, 23.2 mmol). The mixture was stirred at 0 °C for 5 h. The solvent was removed to give the residue, which was purified by flash chromatography (Petroleum ether/Ethyl acetate=20:1 to 3:1) and concentrated to give methyl N-(4-chloropyrimidin-5-yl)carbamate (140 mg, 0.750 mmol, 6.45% yield) as white solid. MS (ESI): m/z = 188.4 [M+H]⁺

### Example C.2

### 4-chloro-3-isopropylbenzonitrile

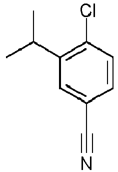

To a solution of 4-amino-3-isopropylbenzonitrile (CAS: 760924-87-6) (2.05 g, 10.9 mmol) in acetonitrile (45 ml) under an inert atmosphere was added copper (II) chloride (1.83 g, 13.6 mmol) and tert-butyl nitrite (1.79 g, 2.07 mL, 17.4 mmol), the reaction mixture was then stirred at room temperature for 20 min followed by 2 h at 60 °C. The reaction was then cooled down to room temperature, diluted with ethyl acetate and extracted with aq. NH₃ 4 M, the organic phase was collected and the aqueous phase was back-extracted with ethyl acetate. The combined organic phases were washed with aq. NaOH 1 M solution, dried over sodium sulfate and evaporated down to dryness to yield 2.05g of a crude oil, which was purified by flash chromatography eluting with a mixture of heptane and ethyl acetate (5% to 30%) to yield 1.22 g of the title compound. MS (ESI): m/z = 179.0 [M+H]⁺

### Example C.3

### tert-butyl N-(4-chloropyrimidin-5-yl)carbamate

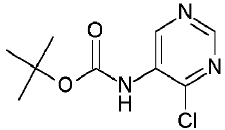

To a solution of 4-chloropyrimidin-5-amine (200 mg, 1.54 mmol,) in THF (2 mL) was added tert-butoxycarbonyl isopropyl carbonate (0.07 mL, 1.54 mmol). The mixture was stirred at 0 °C for 0.5 h. Then the tert-butoxycarbonyl isopropyl carbonate (0.07 mL, 1.54 mmol) was added and stirred for 2 h. The solvent was removed to give the residue, which was purified by flash chromatography (Petroleum ether/Ethyl acetate=20:1 to 3:1 gradient) and concentrated to give tert-butyl N-(4-chloropyrimidin-5-yl)carbamate (98 mg, 0.430 mmol, 27.6 % yield) as white solid. MS (ESI): m/z = 230.1 [M+H]⁺

### Example 54

### Description of the Kv3.1, Kv3.2, Kv3.3, and Kv3.4 assay

In vitro, high-throughput cellular electrophysiology was employed to detect voltage-dependence of compounds in single concentration screening and IC50 determination for library and medicinal chemistry profiling compounds in 384 well plates.

HEK-293 cell lines stably expressing coding DNA for Kv3.1, 3.2, 3.3, and 3.4 were validated on an automated patch clamp (APC) platform, Qube from Sophion/Denmark. Cell cultures were maintained in DMEM cell growth media containing FBS and appropriate antibiotics at 37°C, 5% CO₂ for a maximum of 20 passages. Intra- and extracellular (w. glucose) HEPES buffers containing various monovalent and divalent cations were adjusted to pH 7.2 and 7.4 respectively. Cells were harvested at 75% confluency and resuspended in extracellular buffer and added to wells. Negative pressure was applied to establish whole cell recording by APC.

Currents through activated channels were measured directly by APC by employing depolarization voltage steps. Baseline currents were established for the voltage-dependence protocols using a - 80 mV holding potential and multiple test pulses at +10 mV or by 10 mV steps from -80 mV to +40 mV, respectively. Test compounds were added after baseline recordings followed by a 10 min incubation, and voltage-dependency protocol recordings. Data was corrected for baseline and leak currents (well based) and normalized for % potentiation (plate based). Two previously reported Kv3 positive modulators of varying potencies were used for run to run and plate to plate quality controls. A non-selective and potent positive modulator was used as a positive control. Percent potentiation was analyzed at -20 mV of the voltage-dependency protocol and voltage shift in channel activation was analyzed using the voltage step protocol from -80 mV to + 40 mV. Selectivity was assessed against Kv3.2, Kv3.3, and Kv3.4 overexpressing cell lines using similar methods.

Some exemplary results for compounds of formula (I) are listed in Table 1.

**Table 1**

| **Example** | **Systematic Name** | **EC50 Kv3.1 [µM]** |
|---|---|---|
| 1 | 3-(6-spiro[2H-benzofuran-3,1'-cyclopropane]-4-yloxy-3-pyridyl)-1H-imidazo[4,5-b]pyridin-2-one | 0.3 |
| 2 | 9-(6-spiro[2H-benzofuran-3,1'-cyclopropane]-4-yloxy-3-pyridyl)-7H-purin-8-one | 0.8 |
| 3 | 3-[6-(3-chloro-4-methyl-phenoxy)-3-pyridyl]-1H-imidazo[4,5-b]pyridin-2-one | 1.6 |
| 4 | 3-tert-butyl-4-[[5-(2-oxo-1H-imidazo[4,5-b]pyridin-3-yl)-2-pyridyl]oxy]benzonitrile | 2.0 |
| 5 | 2-isopropyl-4-[[5-(2-oxo-1H-imidazo[4,5-b]pyridin-3-yl)-2-pyridyl]oxy]benzonitrile | 2.7 |
| 6 | 1-(6-spiro[2H-benzofuran-3,1'-cyclopropane]-4-yloxy-3-pyridyl)-3H-imidazo[4,5-c]pyridin-2-one; hydrochloride salt | 2.0 |
| 7 | 6-fluoro-3-[6-(3-methoxy-4-methyl-phenoxy)-3-pyridyl]-1H-imidazo[4,5-b]pyridin-2-one | 2.2 |
| 8 | 3-(3-fluoro-4-spiro[2H-benzofuran-3,1'-cyclopropane]-4-yloxy-phenyl)-1H-imidazo[4,5-b]pyridin-2-one; hydrochloride salt | 2.3 |
| 9 | 3-[6-(3-methoxy-4-methyl-phenoxy)-3-pyridyl]-6-methyl-1H-imidazo[4,5-b]pyridin-2-one | 2.5 |
| 10 | 3-(6-spiro[2H-benzofuran-3,1'-cyclopropane]-4-yloxy-3-pyridyl)-1H-benzimidazol-2-one; formic acid salt | 2.5 |
| 11 | 3-(5-methyl-6-spiro[2H-benzofuran-3,1'-cyclopropane]-4-yloxy-3-pyridyl)-1H-benzimidazol-2-one | 2.8 |
| 12 | 3-(6-spiro[2H-benzofuran-3,1'-cyclopropane]-4-yloxy-3-pyridyl)-1H-imidazo[4,5-c]pyridin-2-one; formic acid salt | 3.9 |
| 13 | 1-(6-spiro[2H-benzofuran-3,1'-cyclopropane]-4-yloxy-3-pyridyl)-3H-imidazo[4,5-b]pyridin-2-one | 3.4 |
| 14 | 4-[[5-(6-fluoro-2-oxo-1H-imidazo[4,5-b]pyridin-3-yl)-2-pyridyl]oxy]-2-isopropyl-benzonitrile | 2.7 |
| 15 | 2-isopropyl-4-[[5-(8-oxo-7H-purin-9-yl)-2-pyridyl]oxy]benzonitrile | 3.5 |
| 16 | 2-isopropyl-4-[[5-(2-oxo-3H-imidazo[4,5-c]pyridin-1-yl)-2-pyridyl]oxy]benzonitrile | 5.9 |
| 17 | 4-[2-fluoro-4-(2-oxo-1H-imidazo[4,5-b]pyridin-3-yl)phenoxy]-2-isopropyl-benzonitrile | 4.7 |
| 18 | 4-[2-fluoro-4-(8-oxo-7H-purin-9-yl)phenoxy]-2-isopropyl-benzonitrile | 18.8 |
| 19 | 2-isopropyl-4-[[5-(2-oxo-3H-benzimidazol-1-yl)-2-pyridyl]oxy]benzonitrile | 15.6 |
| 20 | 3-tert-butyl-4-[[5-(2-oxo-3H-benzimidazol-1-yl)-2-pyridyl]oxy]benzonitrile | 12.2 |
| 21 | 2-ethyl-4-[[5-(2-oxo-3H-benzimidazol-1-yl)-2-pyridyl]oxy]benzonitrile | 18.5 |
| 22 | 2-isopropoxy-4-[[5-(2-oxo-3H-benzimidazol-1-yl)-2-pyridyl]oxy]benzonitrile | 20.7 |
| 23 | 3-(3-fluoro-4-spiro[2H-benzofuran-3,1'-cyclopropane]-4-yloxy-phenyl)-1H-benzimidazol-2-one | 3.5 |
| 24 | 3-tert-butyl-4-[[5-(8-oxo-7H-purin-9-yl)-2-pyridyl]oxy]benzonitrile | 4.0 |
| 25 | 9-[6-[4-methyl-3-(trifluoromethoxy)phenoxy]-3-pyridyl]-7H-purin-8-one | 5.9 |
| 26 | 3-tert-butyl-4-[[5-(2-oxo-3H-imidazo[4,5-c]pyridin-1-yl)-2-pyridyl]oxy]benzonitrile | 4.7 |
| 27 | 3-tert-butyl-4-[[5-(6-fluoro-2-oxo-1H-imidazo[4,5-b]pyridin-3-yl)-2-pyridyl]oxy]benzonitrile | 5.5 |
| 28 | 6-chloro-3-[6-(3-methoxy-4-methyl-phenoxy)-3-pyridyl]-1H-imidazo[4,5-b]pyridin-2-one | 10.0 |
| 29 | 3-[6-(3-methoxy-4-methyl-phenoxy)-3-pyridyl]-6-(trifluoromethyl)-1H-imidazo[4,5-b]pyridin-2-one | 5.7 |
| 30 | 1-[6-(3-methoxy-4-methyl-phenoxy)-3-pyridyl]-3H-imidazo[4,5-b]pyridin-2-one | 19.2 |
| 31 | 3-[6-(3-methoxy-4-methyl-phenoxy)-3-pyridyl]-1H-imidazo[4,5-c]pyridin-2-one | 21.0 |
| 32 | 1-[6-(3-methoxy-4-methyl-phenoxy)-3-pyridyl]-3H-imidazo[4,5-c]pyridin-2-one | 4.4 |
| 33 | 3-[6-(3-methoxy-4-methyl-phenoxy)-3-pyridyl]-1H-imidazo[4,5-b]pyridin-2-one | 3.4 |
| 34 | 3-[6-(3-methoxy-4-methyl-phenoxy)-3-pyridyl]-1H-benzimidazol-2-one | 5.2 |
| 35 | 3-[6-(3-fluoro-4-methyl-phenoxy)-3-pyridyl]-1H-imidazo[4,5-b]pyridin-2-one | 3.6 |
| 36 | 3-[6-(2-fluoro-4-methyl-phenoxy)-3-pyridyl]-1H-imidazo[4,5-b]pyridin-2-one | 4.3 |
| 37 | 3-methyl-4-[[5-(2-oxo-1H-imidazo[4,5-b]pyridin-3-yl)-2-pyridyl]oxy]benzonitrile | 6.0 |
| 38 | 3,5-dimethyl-4-[[5-(2-oxo-1H-imidazo[4,5-b]pyridin-3-yl)-2-pyridyl]oxy]benzonitrile | 5.2 |
| 39 | 3-[6-(2-methoxy-4-methyl-phenoxy)-3-pyridyl]-1H-imidazo[4,5-b]pyridin-2-one | 5.0 |
| 40 | 4-[[5-(2-oxo-1H-imidazo[4,5-b]pyridin-3-yl)-2-pyridyl]oxy]-3-(trifluoromethyl)benzonitrile | 6.6 |
| 41 | 3-[6-[(5-methoxy-6-methyl-3-pyridyl)oxy]-3-pyridyl]-1H-imidazo[4,5-b]pyridin-2-one | 16.4 |
| 42 | 1-[6-(3-chloro-4-methyl-phenoxy)-3-pyridyl]-3H-imidazo[4,5-c]pyridin-2-one | 10.0 |
| 43 | 9-[6-(3-methoxy-4-methyl-phenoxy)-3-pyridyl]-7H-purin-8-one | 7.4 |
| 44 | 1-[5-fluoro-6-(3-methoxy-4-methyl-phenoxy)-3-pyridyl]-3H-imidazo[4,5-c]pyridin-2-one | 2.9 |
| 45 | 1-[5-methyl-6-(3-methoxy-4-methyl-phenoxy)-3-pyridyl]-3H-imidazo[4,5-c]pyridin-2-one | 3.1 |
| 46 | 3-[6-(3-methoxy-4-methyl-phenoxy)-5-methyl-3-pyridyl]-1H-imidazo[4,5-b]pyridin-2-one | 4.0 |
| 47 | 1-[6-[4-methyl-3-(trifluoromethoxy)phenoxy]-3-pyridyl]-3H-imidazo[4,5-c]pyridin-2-one | 2.4 |
| 48 | 3-[6-(3-methoxy-4-methyl-phenoxy)-3-pyridyl]-1H-imidazo[4,5-b]pyrazin-2-one | 3.9 |
| 49 | 2-ethyl-4-[[5-(2-oxo-1H-imidazo[4,5-b]pyridin-3-yl)-2-pyridyl]oxy]benzonitrile; hydrochloride salt | 4.1 |
| 50 | 9-[6-(3-chloro-4-methyl-phenoxy)-3-pyridyl]-7H-purin-8-one | 12.2 |
| 51 | 3-tert-butyl-4-[2-fluoro-4-(2-oxo-1H-imidazo[4,5-b]pyridin-3-yl)phenoxy]benzonitrile | 12.7 |
| 52 | 3-isopropyl-4-[3-(2-oxo-1H-imidazo[4,5-b]pyridin-3-yl)cyclobutoxy]benzonitrile | 17.4 |
| 53 | 1-[3-(3-methoxy-4-methyl-phenoxy)cyclobutyl]-3H-imidazo[4,5-c]pyridin-2-one | 3.6 |

### Example 55

A compound of formula (I) can be used in a manner known per se as the active ingredient for the production of tablets of the following composition:

| | Per tablet |
|---|---|
| Active ingredient | 200 mg |
| Microcrystalline cellulose | 155 mg |
| Corn starch | 25 mg |
| Talc | 25 mg |
| Hydroxypropylmethylcellulose | 20 mg |
| | 425 mg |

### Example 56

A compound of formula (I) can be used in a manner known per se as the active ingredient for the production of capsules of the following composition:

| | Per capsule |
|---|---|
| Active ingredient | 100.0 mg |
| Corn starch | 20.0 mg |
| Lactose | 95.0 mg |
| Talc | 4.5 mg |
| Magnesium stearate | 0.5 mg |
| | 220.0 mg |

## Claims

1. A compound of formula (I) 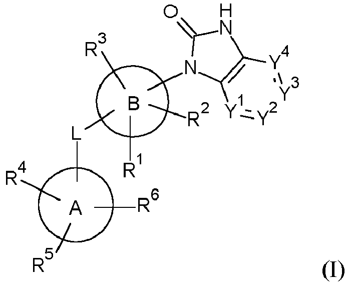 or a pharmaceutically acceptable salt thereof,
wherein:
A and B are each independently selected from C₆-C₁₄-aryl, 5- to 14-membered heteroaryl, C₃-C₁₀-cycloalkyl and 3- to 14-membered heterocyclyl;
L is selected from -CH₂-, -O-, -NH-, -S-, -SO- and -SO₂-;
Y¹, Y², Y³ and Y⁴ are each independently selected from CH, N, C-halogen, C-cyano, C-OH, C-C₁-C₆-alkyl, C-halo-C₁-C₆-alkyl, C-hydroxy-C₁-C₆-alkyl, C-C₁-C₆-alkoxy-C₁-C₆-alkyl, C-C₁-C₆-alkoxy, C-halo-C₁-C₆-alkoxy, C-S-C₁-C₆-alkyl, C-SO-C₁-C₆-alkyl, C-SO₂-C₁-C₆-alkyl, C-C₃-C₁₀-cycloalkyl and C-(3- to 14-membered heterocyclyl);
(i) R¹, R², R³, R⁴, R⁵ and R⁶ are each independently selected from hydrogen, halogen, cyano, hydroxy, C₁-C₆-alkyl, halo-C₁-C₆-alkyl, hydroxy-C₁-C₆-alkyl, C₁-C₆-alkoxy-C₁-C₆-alkyl, C₁-C₆-alkoxy, halo-C₁-C₆-alkoxy, -S-C₁-C₆-alkyl, - SO-C₁-C₆-alkyl, -SO₂-C₁-C₆-alkyl, C₃-C₁₀-cycloalkyl and 3- to 14-membered heterocyclyl; or
(ii) R¹, R², R³, and R⁶ are each independently selected from hydrogen, halogen, cyano, hydroxy, C₁-C₆-alkyl, halo-C₁-C₆-alkyl, hydroxy-C₁-C₆-alkyl, C₁-C₆-alkoxy-C₁-C₆-alkyl, C₁-C₆-alkoxy, halo-C₁-C₆-alkoxy, -S-C₁-C₆-alkyl, -SO-C₁-C₆-alkyl, -SO₂-C₁-C₆-alkyl, C₃-C₁₀-cycloalkyl and 3- to 14-membered heterocyclyl; and
R⁴ and R⁵, taken together with the atoms to which they are attached, form a 3-to 14-membered heterocycle or a C₃-C₁₀-cycloalkyl.

2. The compound of formula (I) according to claim 1, or a pharmaceutically acceptable salt thereof, wherein A is selected from C₆-C₁₄-aryl and 5- to 14-membered heteroaryl.

3. The compound of formula (I) according to claim 1, or a pharmaceutically acceptable salt thereof, wherein A is C₆-C₁₄-aryl.

4. The compound of formula (I) according to claim 1, or a pharmaceutically acceptable salt thereof, wherein A is phenyl.

5. The compound of formula (I) according to any one of claims 1 to 4, or a pharmaceutically acceptable salt thereof, wherein B is selected from C₆-C₁₄-aryl, 5-to 14-membered heteroaryl and C₃-C₁₀-cycloalkyl.

6. The compound of formula (I) according to any one of claims 1 to 4, or a pharmaceutically acceptable salt thereof, wherein B is 5- to 14-membered heteroaryl.

7. The compound of formula (I) according to any one of claims 1 to 4, or a pharmaceutically acceptable salt thereof, wherein B is pyridyl.

8. The compound of formula (I) according to any one of claims 1 to 7, or a pharmaceutically acceptable salt thereof, wherein L is -O-.

9. The compound of formula (I) according to any one of claims 1 to 8, or a pharmaceutically acceptable salt thereof, wherein Y¹ is CH or N.

10. The compound of formula (I) according to any one of claims 1 to 9, or a pharmaceutically acceptable salt thereof, wherein Y² is CH or N.

11. The compound of formula (I) according to any one of claims 1 to 9, or a pharmaceutically acceptable salt thereof, wherein Y² is CH.

12. The compound of formula (I) according to any one of claims 1 to 11, or a pharmaceutically acceptable salt thereof, wherein Y³ is selected from CH, N, C-halogen, C-C₁-C₆-alkyl and C-halo-C₁-C₆-alkyl.

13. The compound of formula (I) according to any one of claims 1 to 11, or a pharmaceutically acceptable salt thereof, wherein Y³ is selected from CH, N, C-halogen and C-C₁-C₆-alkyl.

14. The compound of formula (I) according to any one of claims 1 to 11, or a pharmaceutically acceptable salt thereof, wherein Y³ is selected from CH, N, C-F and C-methyl.

15. The compound of formula (I) according to any one of claims 1 to 14, or a pharmaceutically acceptable salt thereof, wherein Y⁴ is CH or N.

16. The compound of formula (I) according to any one of claims 1 to 14, or a pharmaceutically acceptable salt thereof, wherein Y⁴ is CH.

17. The compound of formula (I) according to any one of claims 1 to 16, or a pharmaceutically acceptable salt thereof, wherein R¹ is selected from hydrogen, halogen and C₁-C₆-alkyl.

18. The compound of formula (I) according to any one of claims 1 to 16, or a pharmaceutically acceptable salt thereof, wherein R¹ is hydrogen.

19. The compound of formula (I) according to any one of claims 1 to 18, or a pharmaceutically acceptable salt thereof, wherein R² is hydrogen.

20. The compound of formula (I) according to any one of claims 1 to 19, or a pharmaceutically acceptable salt thereof, wherein R³ is hydrogen.

21. The compound of formula (I) according to any one of claims 1 to 20, or a pharmaceutically acceptable salt thereof, wherein R⁴ is selected from C₁-C₆-alkyl, halo-C₁-C₆-alkyl and C₁-C₆-alkoxy.

22. The compound of formula (I) according to any one of claims 1 to 20, or a pharmaceutically acceptable salt thereof, wherein R⁴ is C₁-C₆-alkyl.

23. The compound of formula (I) according to any one of claims 1 to 20, or a pharmaceutically acceptable salt thereof, wherein R⁴ is methyl.

24. The compound of formula (I) according to any one of claims 1 to 23, or a pharmaceutically acceptable salt thereof, wherein R⁵ is selected from halogen, cyano, C₁-C₆-alkoxy and halo-C₁-C₆-alkoxy.

25. The compound of formula (I) according to any one of claims 1 to 23, or a pharmaceutically acceptable salt thereof, wherein R⁵ is C₁-C₆-alkoxy.

26. The compound of formula (I) according to any one of claims 1 to 23, or a pharmaceutically acceptable salt thereof, wherein R⁵ is methoxy.

27. The compound of formula (I) according to any one of claims 1 to 20, or a pharmaceutically acceptable salt thereof, wherein R⁴ and R⁵, taken together with the atoms to which they are attached, form a 3- to 14-membered heterocycle.

28. The compound of formula (I) according to any one of claims 1 to 20, or a pharmaceutically acceptable salt thereof, wherein R⁴ and R⁵, taken together with the atoms to which they are attached, form a 5-oxaspiro[2.4]hept-6-ene or a 5-thiaspiro[2.4]hept-6-ene ring.

29. The compound of formula (I) according to any one of claims 1 to 28, or a pharmaceutically acceptable salt thereof, wherein R⁶ is hydrogen or C₁-C₆-alkyl.

30. The compound of formula (I) according to any one of claims 1 to 28, or a pharmaceutically acceptable salt thereof, wherein R⁶ is hydrogen.

31. The compound of formula (I) according to any one of claims 1 to 28, or a pharmaceutically acceptable salt thereof, wherein:
A is selected from C₆-C₁₄-aryl and 5- to 14-membered heteroaryl;
B is selected from C₆-C₁₄-aryl, 5- to 14-membered heteroaryl and C₃-C₁₀-cycloalkyl;
L is -O-;
Y¹, Y² and Y⁴ are each independently selected from CH and N;
Y³ is selected from CH, N, C-halogen, C-C₁-C₆-alkyl and C-halo-C₁-C₆-alkyl;
R¹ is selected from hydrogen, halogen and C₁-C₆-alkyl;
R² and R³ are each hydrogen;
R⁴ is selected from C₁-C₆-alkyl, halo-C₁-C₆-alkyl and C₁-C₆-alkoxy; and
R⁵ is selected from halogen, cyano, C₁-C₆-alkoxy and halo-C₁-C₆-alkoxy; or
R⁴ and R⁵, taken together with the atoms to which they are attached, form a 3- to 14-membered heterocycle; and
R⁶ is hydrogen or C₁-C₆-alkyl.

32. The compound of formula (I) according to any one of claims 1 to 28, or a pharmaceutically acceptable salt thereof, wherein:
A is C₆-C₁₄-aryl;
B is 5- to 14-membered heteroaryl;
L is -O-;
Y¹ is CH or N;
Y² and Y⁴ are both CH;
Y³ is selected from CH, N, C-halogen and C-C₁-C₆-alkyl;
R¹, R², R³ and R⁶ are each hydrogen;
R⁴ is C₁-C₆-alkyl; and
R⁵ is C₁-C₆-alkoxy; or
R⁴ and R⁵, taken together with the atoms to which they are attached, form a 3- to 14-membered heterocycle.

33. The compound of formula (I) according to any one of claims 1 to 28, or a pharmaceutically acceptable salt thereof, wherein:
A is phenyl;
B is pyridyl;
L is -O-;
Y¹ is CH or N;
Y² and Y⁴ are both CH;
Y³ is selected from CH, N, C-F and C-methyl;
R¹, R², R³ and R⁶ are each hydrogen;
R⁴ is methyl; and
R⁵ is methoxy; or
R⁴ and R⁵, taken together with the atoms to which they are attached, form a 5-oxaspiro[2.4]hept-6-ene or a 5-thiaspiro[2.4]hept-6-ene ring.

34. A compound of formula (I) according to claim 1, or a pharmaceutically acceptable salt thereof, selected from:
3-(6-spiro[2H-benzofuran-3,1'-cyclopropane]-4-yloxy-3-pyridyl)-1H-imidazo[4,5-b]pyridin-2-one;
9-(6-spiro[2H-benzofuran-3,1'-cyclopropane]-4-yloxy-3-pyridyl)-7H-purin-8-one;
3-[6-(3-chloro-4-methyl-phenoxy)-3-pyridyl]-1H-imidazo[4,5-b]pyridin-2-one;
3-tert-butyl-4-[[5-(2-oxo-1H-imidazo[4,5-b]pyridin-3-yl)-2-pyridyl]oxy]benzonitrile;
1-(6-spiro[2H-benzofuran-3,1'-cyclopropane]-4-yloxy-3-pyridyl)-3H-imidazo[4,5-c]pyridin-2-one;
6-fluoro-3-[6-(3-methoxy-4-methyl-phenoxy)-3-pyridyl]-1H-imidazo[4,5-b]pyridin-2-one;
3-(3-fluoro-4-spiro[2H-benzofuran-3,1'-cyclopropane]-4-yloxy-phenyl)-1H-imidazo[4,5-b]pyridin-2-one;
1-[6-[4-methyl-3-(trifluoromethoxy)phenoxy]-3-pyridyl]-3H-imidazo[4,5-c]pyridin-2-one;
3-[6-(3-methoxy-4-methyl-phenoxy)-3-pyridyl]-6-methyl-1H-imidazo[4,5-b]pyridin-2-one;
3-(6-spiro[2H-benzofuran-3,1'-cyclopropane]-4-yloxy-3-pyridyl)-1H-benzimidazol-2-one;
2-isopropyl-4-[[5-(2-oxo-1H-imidazo[4,5-b]pyridin-3-yl)-2-pyridyl]oxy]benzonitrile;
4-[[5-(6-fluoro-2-oxo-1H-imidazo[4,5-b]pyridin-3-yl)-2-pyridyl]oxy]-2-isopropyl-benzonitrile;
3-(5-methyl-6-spiro[2H-benzofuran-3,1'-cyclopropane]-4-yloxy-3-pyridyl)-1H-benzimidazol-2-one;
1-[5-fluoro-6-(3-methoxy-4-methyl-phenoxy)-3-pyridyl]-3H-imidazo[4,5-c]pyridin-2-one;
1-[6-(3-methoxy-4-methyl-phenoxy)-5-methyl-3-pyridyl]-3H-imidazo[4,5-c]pyridin-2-one;
3-[6-(3-methoxy-4-methyl-phenoxy)-3-pyridyl]-1H-imidazo[4,5-b]pyridin-2-one;
1-(6-spiro[2H-benzofuran-3,1'-cyclopropane]-4-yloxy-3-pyridyl)-3H-imidazo[4,5-b]pyridin-2-one;
2-isopropyl-4-[[5-(8-oxo-7H-purin-9-yl)-2-pyridyl]oxy]benzonitrile;
3-(3-fluoro-4-spiro[2H-benzofuran-3,1'-cyclopropane]-4-yloxy-phenyl)-1H-benzimidazol-2-one;
1-[3-(3-methoxy-4-methyl-phenoxy)cyclobutyl]-3H-imidazo[4,5-c]pyridin-2-one;
3-[6-(3-fluoro-4-methyl-phenoxy)-3-pyridyl]-1H-imidazo[4,5-b]pyridin-2-one;
3-(6-spiro[2H-benzofuran-3,1'-cyclopropane]-4-yloxy-3-pyridyl)-1H-imidazo[4,5-c]pyridin-2-one;
3-[6-(3-methoxy-4-methyl-phenoxy)-3-pyridyl]-1H-imidazo[4,5-b]pyrazin-2-one;
3-tert-butyl-4-[[5-(8-oxo-7H-purin-9-yl)-2-pyridyl]oxy]benzonitrile;
3-[6-(3-methoxy-4-methyl-phenoxy)-5-methyl-3-pyridyl]-1H-imidazo[4,5-b]pyridin-2-one;
2-ethyl-4-[[5-(2-oxo-1H-imidazo[4,5-b]pyridin-3-yl)-2-pyridyl]oxy]benzonitrile;
3-[6-(2-fluoro-4-methyl-phenoxy)-3-pyridyl]-1H-imidazo[4,5-b]pyridin-2-one;
1-[6-(3-methoxy-4-methyl-phenoxy)-3-pyridyl]-3H-imidazo[4,5-c]pyridin-2-one;
4-[2-fluoro-4-(2-oxo-1H-imidazo[4,5-b]pyridin-3-yl)phenoxy]-2-isopropyl-benzonitrile;
3-tert-butyl-4-[[5-(2-oxo-3H-imidazo[4,5-c]pyridin-1-yl)-2-pyridyl]oxy]benzonitrile;
3-[6-(2-methoxy-4-methyl-phenoxy)-3-pyridyl]-1H-imidazo[4,5-b]pyridin-2-one;
3,5-dimethyl-4-[[5-(2-oxo-1H-imidazo[4,5-b]pyridin-3-yl)-2-pyridyl]oxy]benzonitrile;
3-[6-(3-methoxy-4-methyl-phenoxy)-3-pyridyl]-1H-benzimidazol-2-one;
3-tert-butyl-4-[[5-(6-fluoro-2-oxo-1H-imidazo[4,5-b]pyridin-3-yl)-2-pyridyl]oxy]benzonitrile;
3-[6-(3-methoxy-4-methyl-phenoxy)-3-pyridyl]-6-(trifluoromethyl)-1H-imidazo[4,5-b]pyridin-2-one;
9-[6-[4-methyl-3-(trifluoromethoxy)phenoxy]-3-pyridyl]-7H-purin-8-one;
2-isopropyl-4-[[5-(2-oxo-3H-imidazo[4,5-c]pyridin-1-yl)-2-pyridyl]oxy]benzonitrile;
3-methyl-4-[[5-(2-oxo-1H-imidazo[4,5-b]pyridin-3-yl)-2-pyridyl]oxy]benzonitrile;
4-[[5-(2-oxo-1H-imidazo[4,5-b]pyridin-3-yl)-2-pyridyl]oxy]-3-(trifluoromethyl)benzonitrile;
9-[6-(3-methoxy-4-methyl-phenoxy)-3-pyridyl]-7H-purin-8-one;
6-chloro-3-[6-(3-methoxy-4-methyl-phenoxy)-3-pyridyl]-1H-imidazo[4,5-b]pyridin-2-one;
1-[6-(3-chloro-4-methyl-phenoxy)-3-pyridyl]-3H-imidazo[4,5-c]pyridin-2-one;
3-tert-butyl-4-[[5-(2-oxo-3H-benzimidazol-1-yl)-2-pyridyl]oxy]benzonitrile;
9-[6-(3-chloro-4-methyl-phenoxy)-3-pyridyl]-7H-purin-8-one;
3-tert-butyl-4-[2-fluoro-4-(2-oxo-1H-imidazo[4,5-b]pyridin-3-yl)phenoxy]benzonitrile;
2-isopropyl-4-[[5-(2-oxo-3H-benzimidazol-1-yl)-2-pyridyl]oxy]benzonitrile;
3-[6-[(5-methoxy-6-methyl-3-pyridyl)oxy]-3-pyridyl]-1H-imidazo[4,5-b]pyridin-2-one;
3-isopropyl-4-[3-(2-oxo-1H-imidazo[4,5-b]pyridin-3-yl)cyclobutoxy]benzonitrile;
2-ethyl-4-[[5-(2-oxo-3H-benzimidazol-1-yl)-2-pyridyl]oxy]benzonitrile;
4-[2-fluoro-4-(8-oxo-7H-purin-9-yl)phenoxy]-2-isopropyl-benzonitrile;
1-[6-(3-methoxy-4-methyl-phenoxy)-3-pyridyl]-3H-imidazo[4,5-b]pyridin-2-one;
2-isopropoxy-4-[[5-(2-oxo-3H-benzimidazol-1-yl)-2-pyridyl]oxy]benzonitrile; and
3-[6-(3-methoxy-4-methyl-phenoxy)-3-pyridyl]-1H-imidazo[4,5-c]pyridin-2-one.

35. A compound of formula (I) according to claim 1, or a pharmaceutically acceptable salt thereof, selected from:
3-(6-spiro[2H-benzofuran-3,1'-cyclopropane]-4-yloxy-3-pyridyl)-1H-imidazo[4,5-b]pyridin-2-one;
9-(6-spiro[2H-benzofuran-3,1'-cyclopropane]-4-yloxy-3-pyridyl)-7H-purin-8-one;
1-(6-spiro[2H-benzofuran-3,1'-cyclopropane]-4-yloxy-3-pyridyl)-3H-imidazo[4,5-c]pyridin-2-one;
6-fluoro-3-[6-(3-methoxy-4-methyl-phenoxy)-3-pyridyl]-1H-imidazo[4,5-b]pyridin-2-one;
3-[6-(3-methoxy-4-methyl-phenoxy)-3-pyridyl]-6-methyl-1H-imidazo[4,5-b]pyridin-2-one;
3-[6-(3-methoxy-4-methyl-phenoxy)-3-pyridyl]-1H-imidazo[4,5-b]pyridin-2-one; and
1-[6-(3-methoxy-4-methyl-phenoxy)-3-pyridyl]-3H-imidazo[4,5-c]pyridin-2-one.

36. A process for manufacturing the compounds of formula (I) according to any one of claims 1 to 35, or pharmaceutically acceptable salts thereof, comprising reacting a diamine of formula **1** 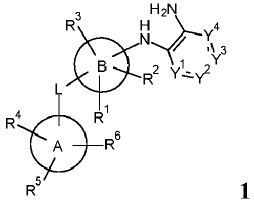 with a coupling agent, to form said compound of formula (I).

37. A compound of formula (I) according to any one of claims 1 to 35, or a pharmaceutically acceptable salt thereof, when manufactured according to the process of claim 36.

38. A compound of formula (I) according to any one of claims 1 to 35 and 37, or a pharmaceutically acceptable salt thereof, for use as therapeutically active substance.

39. A pharmaceutical composition comprising a compound of formula (I) according to any one of claims 1 to 35 and 37, or a pharmaceutically acceptable salt thereof, and a therapeutically inert carrier.

40. A compound of formula (I) according to any one of claims 1 to 35 and 37, or a pharmaceutically acceptable salt thereof, or a pharmaceutical composition according to claim 39, for use in the treatment or prophylaxis of Kv3 mediated diseases and disorders.

41. The compound for use or the pharmaceutical composition for use according to claim 40, wherein said Kv3 mediated diseases and disorders are neurodevelopmental disorders.

42. The compound for use or the pharmaceutical composition for use according to claim 41, wherein said neurodevelopmental disorders are selected from the group consisting of autism, fragile X syndrome, epilepsy, intellectual disability, cognitive impairment, ataxia, depression, schizophrenia, attention deficit hyperactivity disorder and sensory processing disorders (e.g., auditory sensory processing disorders).

43. The invention as described hereinbefore.
